# EUROPEAN PATENT APPLICATION

(11) **EP 4 745 157 A1**
(43) Date of publication of application: **20.05.2026**
(21) Application number: 24843449.0
(22) Date of filing: 12.07.2024
(51) Int. Cl.: C07K 16/28, A61K 31/4412, A61K 45/06, A61P 35/00, G01N 33/574, A61K 39/00

(54) **NOVEL TM4SF5-SPECIFIC ANTIBODY**

(30) Priority: 14.07.2023 KR 20230091977
(71) Applicant: Korea Research Institute of Bioscience and Biotechnology, Daejeon 34141 (KR); Seoul National University R&DB Foundation, Seoul 08826 (KR)
(72) Inventor: KIM, Semi, Daejeon 34141 (KR); LEE, Jung Weon, Seoul 06624 (KR); KIM, Eunmi, Seoul 06262 (KR); KO, Dongjoon, Daejeon 34141 (KR); YOON, Junghwa, Daejeon 34141 (KR)
(74) Representative: Regimbeau
(86) International application number: PCT/KR2024/010060
(87) International publication number: WO 2025/018733

(57) **Abstract**

The present invention relates to a TM4SF5-specific antibody and use thereof.

## Description

### [Technical Field]

The present invention relates to a TM4SF5-specific antibody and use thereof.

### [Background Art]

Transmembrane 4 superfamily (TM4SF) proteins are a group of hydrophobic proteins having a molecular weight of about 25 kDa to 50 kDa, including four transmembrane domains, two extracellular loops, and two short cytoplasmic tail regions, which are also called tetraspanin or tetraspan. The TM4SF proteins form a complex on the cell membrane along with cell adhesion molecules (e.g., integrins), thereby establishing a gigantic tetraspanin-enriched microdomain (TERM) and contributing to various biological functions (e.g., cell adhesion, proliferation, differentiation, metabolic control, interactions with the immune system, and migration).

TM4SF5, which refers to transmembrane 4 L6 family member 5 or four-transmembrane L6 superfamily member 5, is a member of the tetraspanin family, and has a structure including four domains of non-soluble proteins which penetrate through cell membranes, with two loops present extracellularly, and one loop and two tails present in the cytoplasm. TM4SF5 is a homologue of the tumor-associated antigen L6 (TM4SF1), and mRNA of TM4SF1 is known to be highly overexpressed in pancreatic cancer, stomach cancer, liver cancer, colorectal cancer, soft tissue sarcoma, *etc.* Additionally, artificial expression of a TM4SF5 protein in COS7 cells resulted in actin reorganization and focal adhesion turnover, thus suggesting that TM4SF5 may be involved in cell migration through epithelial-mesenchymal transition (EMT) (Lee S. A. et al., J Clin Invest 2008, 118(4):1354-66). Additionally, TM4SF5 proteins, due to their high amino acid sequence homology to L6 (*i.e*., a cancer-related gene), have been suspected of being encoded by a cancer-related gene, and have been reported to be associated with the development and progression of cancer. TM4SF5 is involved in cell proliferation by promoting the progression of the G1/S cycle through the intracellular expression of p27Kip1 and the activity of RhoA GTPase (Kim H. et al., Biochim Biophys Acta 2010 1803(8):975-82), and the cross-talk in the signaling pathway between transforming growth factor-β1 (TGF-β1) and epidermal growth factor receptor (EGFR) involved in EMT is known to induce the expression of TM4SF5, thereby bringing about EMT (Kang M. et al., Biochem J 2012 443(3):691-700).

### [Disclosure]

### [Technical Problem]

As described above, with the emerging importance of TM4SF5 as a specific protein and anticancer target for new cancer diagnosis, studies have been conducted on methods of diagnosing cancer having TM4SF5 as a target. Additionally, for cancer treatment with TM4SF5 as a target, studies have been conducted on the inhibition of the biological activities of TM4SF5 in various fields. In particular, studies have been conducted on compounds that can inhibit the activities of TM4SF5, for example, sulfonamide- and sulfonate-substituted chalcone derivatives have been reported to inhibit the biological activities of TM4SF5 (KR Patent No. 10-0934706). In addition to the above compounds that inhibit the biological activity of TM4SF5, the importance of developing antibody therapeutic agents that specifically bind to TM4SF5 has also been highlighted.

Accordingly, the present inventors have developed a humanized antibody that specifically binds to human TM4SF5 protein and further improved the same, thereby completing the present invention.

### [Technical Solution]

An object of the present invention is to provide a TM4SF5-binding antibody or antigen-binding fragment thereof.

Another object of the present invention is to provide a pharmaceutical composition for preventing or treating a TM4SF5-related disease, comprising the antibody or antigen-binding fragment thereof.

Still another object of the present invention is to provide a composition for diagnosing a TM4SF5-related disease, comprising the antibody or antigen-binding fragment thereof.

Still another object of the present invention is to provide a polynucleotide encoding the antibody or antigen-binding fragment thereof and a transformant comprising the polynucleotide.

### [Advantageous Effects]

The antibody of the present invention may be usefully employed for the prevention, treatment, and diagnosis of a disease in which the TM4SF5 protein is overexpressed.

### [Brief Description of the Drawings]

FIG. 1 and FIG. 2 show the results confirming physicochemical properties of huAb27-8, huAb27-9, huAb27-43, and huAb27-69 antibodies produced using the same vector and expression system as a chimeric antibody. (FIGS. 1A and 2A: SDS-PAGE; FIGS. 1B and 2B: SE-HPLC)
FIG. 3 shows the protein analysis results of huAb27-8 scFv-Fc and huAb27-9 scFv-Fc antibodies through SDS-PAGE and SE-HPLC analyses performed under reducing and non-reducing conditions.
FIG. 4 shows the results confirming the binding ability of huAb27-8, huAb27-9, huAb27-43, and huAb27-69 antibodies produced using the same vector and expression system as the chimeric antibody.
FIG. 5 shows the amino acid sequence of huAb27-9 scFv-Fc antibody.
FIG. 6 illustrates an immunogenicity prediction method.
FIG. 7 and FIG. 8 respectively show the immunogenicity prediction results of VH and VL regions of a humanized antibody.
FIG. 9 shows the results confirming the affinity of Ab27 scFv-Fc and humanized Ab27-9 scFv-Fc through SPR analysis.
FIG. 10 shows the results confirming the cytotoxicity of humanized antibodies against SNU398 cells and HCT116 cells.
FIG. 11 illustrates the process of affinity optimization using untargeted mutagenesis.
FIG. 12 shows the panning results of an error-prone PCR library.
FIG. 13 shows the sequence analysis results of clones selected through screening.
FIG. 14 shows the results of measuring (ELISA) the binding affinity of clones selected through screening.
FIG. 15 shows the SDS-PAGE and SE-HPLC results of selected antibody E01.
FIG. 16 shows the affinity measurement results (Biacore) of the selected antibody E01.
FIG. 17 shows target sites for CDR randomization.
FIG. 18 shows the panning results of a CDR-randomized library.
FIG. 19 shows the sequence analysis results of clones selected through screening.
FIG. 20 shows the affinity measurement results of selected antibodies.
FIG. 21 shows the screening results of a combinatorial library.
FIG. 22 shows the sequence analysis results of clones selected through combinatorial library screening.
FIG. 23 shows the affinity measurement results (Biacore) of antibodies selected through combinatorial library screening.
FIG. 24 shows the ELISA binding analysis results of six clones (T1-5, T1-6, T1-7, T1-8, T2-1, and T2-3).
FIG. 25 shows the analysis results of the binding affinity of six antibodies produced as IgG antibodies against hEC2-Fc-myc antigen, measured by ELISA and Octet.
FIG. 26 shows the results of measuring the cell-surface binding ability of EO1, T35, S3, S13, and S43 antibodies through FACS and IF assays.
FIG. 27 shows the results of measuring the cell-surface binding ability of EO1 and S43 antibodies with an added glycine in the elbow region through FACS and IF assays.
FIG. 28 shows the results of measuring the K_{D} values of EO1 and S43 antibodies with an added glycine in the elbow region by using a Biacore instrument.
FIG. 29 shows the results of analyzing the internalization level of EO1 and S43 antibodies with an added glycine in the elbow region.
FIG. 30 shows the results confirming the cytotoxicity of EO1 and S43 antibodies with an added glycine in the elbow region against SNU398 cells.
FIG. 31 shows the results confirming the cytotoxicity of EO1 and S43 antibodies with an added glycine in the elbow region against SNU398 cells by using a soft agar assay.
FIG. 32 shows the results confirming the *in vivo* tumor inhibition activity of Ab27 scFv-Fc (300 µg), Hz #9 H(G)L(GG), EO1 H(G)L(GG), and S43 IgG antibodies in an SNU398 xenograft model.
FIG. 33 and FIG. 34 show the results confirming the cell-surface binding ability of clones 1-5, 1-6, 1-7, 1-8, 2-1, and 2-3 through FACS and IF assays.
FIG. 35 shows the results confirming the K_{D} values of clones 1-5, 1-6, 1-7, 1-8, and 2-1 by using a BIACORE instrument.
FIG. 36 shows the purification results of selected clones S43, 1-5, and 2-1.
FIG. 37 shows the SPR analysis results of S43, 1-5, and 2-1.
FIG. 38 shows the results confirming the cell-surface binding ability of S43, 1-5, and 2-1 through FACS and IF assays.
FIGS. 39 and 40 show the internalization assay results of S43, 1-5, and 2-1. FIG. 39 shows FACS analysis of the remaining antibody bound on the surface of Tp cells at 0, 2, 4, and 6 hours, and FIG. 40 shows the results of conducting a Co-IF experiment with the lysosome marker LysoTracker in order to track the movement of TM4SF5 antibody to the lysosome after internalization.
FIG. 41 shows the results confirming the ability of S43, 1-5, and 2-1 to inhibit the growth of the SNU398 liver cancer cell line.
FIG. 42 confirms the tumor inhibition ability of S43, 1-5, and 2-1 through soft agar assay.
FIG. 43 confirms the effects of the administration of S43, 1-5, and 2-1 in combination with sorafenib or cetuximab.
FIG. 44 shows the results confirming the ability of 1-5 to inhibit tumor metastasis *in vivo.*
FIG. 45 shows the results confirming the antitumor efficacy in an SNU398 cell xenograft mouse model.
FIG. 46 shows the results of evaluating the antitumor efficacy of antibodies 1-5 and 2-1 in an SNU449 T7 xenograft model.
FIG. 47 shows the results confirming the antitumor efficacy in an HT-29 cell xenograft mouse model.
FIG. 48 shows the results of evaluating the *in vivo* tumor-targeting capability by conjugating a fluorescent dye to the antibody.

### [Detailed Description of Preferred Embodiments]

An aspect of the present invention provides a TM4SF5-binding antibody or antigen-binding fragment thereof.

In a specific embodiment, the antibody or antigen-binding fragment thereof comprises a variable domain comprising CDRs selected from the following:
(i) a heavy chain variable domain (VH) comprising HCDR1 having an amino acid sequence of SEQ ID NO: 16, HCDR2 having an amino acid sequence of SEQ ID NO: 17, and HCDR3 having an amino acid sequence of SEQ ID NO: 18, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13;
(ii) a heavy chain variable domain (VH) comprising HCDR1 having an amino acid sequence of SEQ ID NO: 24, HCDR2 having an amino acid sequence of SEQ ID NO: 25, and HCDR3 having an amino acid sequence of SEQ ID NO: 26, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13;
(iii) a heavy chain variable domain (VH) comprising HCDR1 having an amino acid sequence of SEQ ID NO: 32, HCDR2 having an amino acid sequence of SEQ ID NO: 33, and HCDR3 having an amino acid sequence of SEQ ID NO: 34, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13; and
(iv) a heavy chain variable domain (VH) comprising HCDR1 having an amino acid sequence of SEQ ID NO: 40, HCDR2 having an amino acid sequence of SEQ ID NO: 41, and HCDR3 having an amino acid sequence of SEQ ID NO: 42, and a light chain variable domain (VL) comprising LCDR1 having an amino acid sequence of SEQ ID NO: 48, LCDR2 having an amino acid sequence of SEQ ID NO: 49, and LCDR3 having an amino acid sequence of SEQ ID NO: 50,
wherein the CDR sequences are designated according to the Kabat numbering system.

The antibody or binding fragment thereof according to any one of the foregoing specific embodiments comprises a heavy chain variable domain (VH) comprising HCDR1 having the amino acid sequence of SEQ ID NO: 16, HCDR2 having the amino acid sequence of SEQ ID NO: 17, and HCDR3 having the amino acid sequence of SEQ ID NO: 18, and a light chain variable domain (VL) comprising LCDR1 having the amino acid sequence of SEQ ID NO: 11, LCDR2 having the amino acid sequence of SEQ ID NO: 12, and LCDR3 having the amino acid sequence of SEQ ID NO: 13.

The antibody or binding fragment thereof according to any one of the foregoing specific embodiments comprises a heavy chain variable domain (VH) comprising HCDR1 having the amino acid sequence of SEQ ID NO: 24, HCDR2 having the amino acid sequence of SEQ ID NO: 25, and HCDR3 having the amino acid sequence of SEQ ID NO: 26, and a light chain variable domain (VL) comprising LCDR1 having the amino acid sequence of SEQ ID NO: 11, LCDR2 having the amino acid sequence of SEQ ID NO: 12, and LCDR3 having the amino acid sequence of SEQ ID NO: 13.

The antibody or binding fragment thereof according to any one of the foregoing specific embodiments comprises a heavy chain variable domain (VH) comprising HCDR1 having the amino acid sequence of SEQ ID NO: 32, HCDR2 having the amino acid sequence of SEQ ID NO: 33, and HCDR3 having the amino acid sequence of SEQ ID NO: 34, and a light chain variable domain (VL) comprising LCDR1 having the amino acid sequence of SEQ ID NO: 11, LCDR2 having the amino acid sequence of SEQ ID NO: 12, and LCDR3 having the amino acid sequence of SEQ ID NO: 13.

The antibody or binding fragment thereof according to the foregoing specific embodiments comprises a heavy chain variable domain (VH) comprising HCDR1 having the amino acid sequence of SEQ ID NO: 40, HCDR2 having the amino acid sequence of SEQ ID NO: 41, and HCDR3 having the amino acid sequence of SEQ ID NO: 42, and a light chain variable domain (VL) comprising LCDR1 having the amino acid sequence of SEQ ID NO: 48, LCDR2 having the amino acid sequence of SEQ ID NO: 49, and LCDR3 having the amino acid sequence of SEQ ID NO: 50.

The antibody or binding fragment thereof according to any one of the foregoing specific embodiments comprises a heavy chain variable domain (VH) comprising HCDR1 having the amino acid sequence of SEQ ID NO: 19 or 27, HCDR2 having the amino acid sequence of SEQ ID NO: 20 or 28, and HCDR3 having the amino acid sequence of SEQ ID NO: 5, defined by Chothia numbering system.

The antibody or antigen-binding fragment thereof according to any one of the foregoing specific embodiments comprises a heavy chain variable domain having an amino acid sequence selected from SEQ ID NOS: 14, 22, and 30, and a light chain variable domain having the amino acid sequence of SEQ ID NO: 9.

The antibody or antigen-binding fragment thereof according to any one of the foregoing specific embodiments comprises a heavy chain variable domain (VH) having the amino acid sequence of SEQ ID NO: 38, and a light chain variable domain (VL) having the amino acid sequence of SEQ ID NO: 46.

The antibody or binding fragment thereof according to any one of the foregoing specific embodiments is selected from the group consisting of Fab fragments, Fab' fragments, F'(ab)2 fragments, Fd fragments, Fv fragments, diabodies, biparatopic peptides, domain antibodies (dAbs), CDR-grafted antibodies, single chain antibodies (scFv), scFv-Fc, scFv-zippers, scFab, single chain antibody fragments, diabodies, triabodies, tetrabodies, minibodies, linear antibodies, chelated recombinant antibodies, tribodies, bibodies, intrabodies, nanobodies, small modular immunopharmaceuticals (SMIPs), antigen binding domain immunoglobulin fusion proteins, single domain antibodies, and antibodies containing VHH.

The antibody or antigen-binding fragment thereof according to any one of the foregoing specific embodiments is IgG or scFv-Fc.

Another aspect of the present invention provides a pharmaceutical composition for preventing or treating a TM4SF5-related disease, comprising a TM4SF5-binding antibody or antigen-binding fragment thereof.

In a specific embodiment, the TM4SF5-related disease is selected from fatty liver, liver fibrosis, liver cancer, stomach cancer, colorectal cancer, rectal cancer, oral cancer, pharynx cancer, larynx cancer, lung cancer, colon cancer, breast cancer, cervical cancer, endometrial cancer, ovarian cancer, prostate cancer, testicular cancer, bladder cancer, kidney cancer, pancreatic cancer, bone cancer, connective tissue cancer, skin cancer, brain cancer, thyroid cancer, leukemia, Hodgkin's disease, soft tissue sarcoma, lymphoma, and multiple myeloma hematologic cancer.

In another specific embodiment, the TM4SF5-related disease is selected from fatty liver, liver fibrosis, liver cancer, stomach cancer, colorectal cancer, colon cancer, prostate cancer, pancreatic cancer, and soft tissue sarcoma.

The pharmaceutical composition according to any one of the previous specific embodiments is administered in combination with a substance selected from the group consisting of 5-fluorouracil (5-FU), oxaliplatin, doxorubicin, sorafenib, and cetuximab.

Still another aspect of the present invention provides a method for preventing or treating a TM4SF5-related disease, comprising administering a TM4SF5-binding antibody or antigen-binding fragment thereof to a subject.

Still another aspect of the present invention provides a polynucleotide encoding a TM4SF5-binding antibody or antigen-binding fragment thereof.

Still another aspect of the present invention provides an expression vector comprising a polynucleotide encoding a TM4SF5-binding antibody or antigen-binding fragment thereof.

Still another aspect of the present invention provides a transformant comprising: a polynucleotide encoding a TM4SF5-binding antibody or antigen-binding fragment thereof; or an expression vector comprising the same.

Still another aspect of the present invention provides a composition for diagnosing a TM4SF5-related disease, comprising a TM4SF5-binding antibody or antigen-binding fragment thereof.

Still another aspect of the present invention provides a method for diagnosing a TM4SF5-related disease, comprising detecting a transmembrane 4 L six family member 5 (TM4SF5) protein in a biological sample through an antigen-antibody reaction using a TM4SF5-binding antibody or antigen-binding fragment thereof.

The present invention is described in detail as follows. Meanwhile, respective descriptions and embodiments disclosed in the present invention may also be applied to other descriptions and embodiments. That is, all combinations of various elements disclosed in the present invention fall within the scope of the present invention. Further, the scope of the present invention is not limited by the specific description below.

Additionally, those skilled in the art will be able to recognize or confirm, based on routine experimentation, many equivalents to the specific embodiments of the present invention described in this application, and such equivalents are intended to be included in the present invention.

Additionally, a number of literature references and patent documents are referenced throughout this specification and their citations are indicated. The disclosures of the cited literature references and patent documents are incorporated by reference into this specification in their entirety, so that the level of the technical field to which the present invention belongs and the content of the present invention are more clearly described.

As used herein, the term "antibody" refers to a protein molecule acting as a ligand that specifically recognizes an antigen, including an immunoglobulin molecule having immunological reactivity to a specific antigen.

Additionally, the term also includes bispecific molecules (e.g., bispecific antibodies), diabodies, triabodies, and tetrabodies. A whole antibody has two full-length light chains (LC) and two full-length heavy chains (HC), and each light chain is linked to a heavy chain via a disulfide bond.

The antibody of the present invention includes IgA, IgD, IgE, IgM, and IgG, and IgG includes IgG1, IgG2, IgG3, and IgG4 as subtypes. In an embodiment, the antibody provided in the present invention may be an IgG antibody.

As used herein, the terms "antigen-binding fragment" and "antibody fragment" refer to any fragment of an antibody of the present invention that retains antigen-binding activity and are used interchangeably.

The antibody or antigen binding fragment thereof of the present invention may be selected from the group consisting of Fab fragments, Fab' fragments, F'(ab)2 fragments, Fd fragments, Fv fragments, single chain antibodies, diabodies, biparatopic peptides, domain antibodies (dAbs), CDR-grafted antibodies, single chain antibodies (scFv), single chain antibody fragments, diabodies, triabodies, tetrabodies, minibodies, linear antibodies, chelated recombinant antibodies, tribodies, bibodies, intrabodies, nanobodies, small modular immunopharmaceuticals (SMIPs), antigen binding domain immunoglobulin fusion proteins, single domain antibodies, and antibodies containing VHH. In an embodiment, the antibody provided in the present invention may be an scFv antibody.

The Fd refers to a heavy chain portion comprised in a Fab fragment. The Fab has a structure comprising a variable domain of light and heavy chains, a constant region of a light chain (framework region; FR), and a first constant region of a heavy chain (CH1 domain), and it has one antigen-binding site. A Fab' differs from Fab in that it has a hinge region comprising at least one cysteine residue at the C-terminus of the heavy chain CH1 domain. An F(ab')2 antibody is produced by forming a disulfide bond between cysteine residues in the hinge regions of Fab'. A variable fragment (Fv) refers to a minimum antibody fragment having only a heavy chain variable domain and a light chain variable domain. A double chain Fv (dsFv) has a structure where a heavy chain variable domain and a light chain variable domain are linked via a disulfide bond, and a single chain Fv (scFv) has a structure where a heavy chain variable domain and a light chain variable domain are linked via a covalent bond, generally a peptide linker. These antibody fragments can be obtained using a protease (e.g., Fab fragments can be obtained by cleaving a whole antibody with papain, whereas F(ab')2 fragments can be obtained by cleaving a whole antibody with pepsin), for example, through genetic recombination technology.

For example, the antibody of the present invention may be a humanized antibody.

As used herein, the term "humanized antibody" refers to an antibody prepared in a form where part or the entirety of a CDR sequence of a mouse monoclonal antibody is grafted to a human antibody. For example, the humanized antibody may be obtained by preparing a humanized variable domain by the recombination of the CDRs of a mouse monoclonal antibody with human antibody-derived FRs, followed by the recombination of the same with a constant region of a desirable human antibody.

For example, the antibody of the present invention may include a modification in the elbow region.

As used herein, the term "elbow region" refers to a joint between a variable domain and a constant region in heavy chains and light chains of an antibody. The C-terminus of the variable domain (VH or VL) is directly linked to the N-terminus of the most N-terminal constant domain (generally CH1 or CL), and the junction between the C-terminus of the variable domain (VH or VL) and the N-terminus of the most N-terminal constant domain (generally CH1 or CL) is referred to as the "elbow" or "elbow region".

For example, the antibody of the present invention may include a modification in which glycine is added to the elbow region of a heavy chain and/or light chain, and, for example, may include a modification in which one or two glycine residues are added. However, it is not necessarily limited thereto.

In the present invention, the variable domain of a heavy chain may be designated as "VH"; the variable domain of a light chain may be designated as "VL; the CDR of a heavy chain may each be designated as "VH-CDR1", "VH-CDR2", and "VH-CDR3"; the CDR of a light chain may each be designated as "VL-CDR1", "VL-CDR2", and "VL-CDR3"; the FR of a heavy chain may each be designated as "VH-FR1", "VH-FR2", "VH-FR3", and "VH-FR4"; and the FR of a light chain may each be designated as "VL-FR1", "VL-FR2", "VL-FR3", and "VL-FR4".

The variable domains of a light chain and a heavy chain comprise three complementarity-determining regions (hereinafter, "CDR") and four framework regions (FR). The CDRs mainly serve to bind to the epitope of an antigen. The CDRs of each chain are typically referred to as CDR1, CDR2, and CDR3 sequentially from the N-terminus, and are also distinguished by the chain in which a specific CDR is located.

As used herein, the term "CDR" or "complementarity-determining region" refers to a non-contiguous antigen binding site found within the variable domains of both heavy and light chain polypeptides. Since the number of amino acids in the CDRs of antibodies differs depending on the antibody, it is necessary to assign numbers, starting from the N-terminus based on a fixed rule, to the amino acids in the conserved amino acid sequences (for example, framework regions) and in the variable domains for numerous types of individual VH or VL. Representative examples include the Kabat, Chothia, and IMGT numbering systems, and each differs in the order in which the amino acids in the CDRs are numbered. The Kabat, Chothia, and IMGT numbering systems are described in literature references (Kabat et al., J. Biol. Chem. 252, 6609-6616 (1977); Kabat et al., Sequences of protein of immunological interest. (1991); Chothia et al., J. Mol. Biol. 196:901-917 (1987); and MacCallum et al., J. Mol. Biol. 262:732-745 (1996)), which are incorporated herein by reference in their entirety. The definition above includes overlaps or subsets of amino acid residues when compared with one another. HCDR1, HCDR2, and HCDR3 represent heavy chain CDRs, and LCDR1, LCDR2, and LCDR3 represent light chain CDRs.

In addition to the above references, the CDRs can be defined according to the Kabat, Chothia, or IMGT numbering system by using publicly available databases such as http://cao.labshare.cn/AbRSA/abrsa.php.

In one embodiment, the antibody or binding fragment thereof of the present invention comprises a heavy chain variable domain comprising HCDR1 having the amino acid sequence of SEQ ID NO: 16, HCDR2 having the amino acid sequence of SEQ ID NO: 17, and HCDR3 having the amino acid sequence of SEQ ID NO: 18, and a light chain variable domain comprising LCDR1 having the amino acid sequence of SEQ ID NO: 11, LCDR2 having the amino acid sequence of SEQ ID NO: 12, and LCDR3 having the amino acid sequence of SEQ ID NO: 13.

In one embodiment, the antibody or binding fragment thereof of the present invention comprises a heavy chain variable domain comprising HCDR1 having the amino acid sequence of SEQ ID NO: 24, HCDR2 having the amino acid sequence of SEQ ID NO: 25, and HCDR3 having the amino acid sequence of SEQ ID NO: 26, and a light chain variable domain comprising LCDR1 having the amino acid sequence of SEQ ID NO: 11, LCDR2 having the amino acid sequence of SEQ ID NO: 12, and LCDR3 having the amino acid sequence of SEQ ID NO: 13.

In one embodiment, the antibody or antigen-binding fragment thereof of the present invention comprises a heavy chain variable domain comprising HCDR1 having the amino acid sequence of SEQ ID NO: 32, HCDR2 having the amino acid sequence of SEQ ID NO: 33, and HCDR3 having the amino acid sequence of SEQ ID NO: 34, and a light chain variable domain comprising LCDR1 having the amino acid sequence of SEQ ID NO: 11, LCDR2 having the amino acid sequence of SEQ ID NO: 12, and LCDR3 having the amino acid sequence of SEQ ID NO: 13.

In one embodiment, the antibody or antigen-binding fragment thereof of the present invention comprises a heavy chain variable domain comprising HCDR1 having the amino acid sequence of SEQ ID NO: 40, HCDR2 having the amino acid sequence of SEQ ID NO: 41, and HCDR3 having the amino acid sequence of SEQ ID NO: 42, and a light chain variable domain comprising LCDR1 having the amino acid sequence of SEQ ID NO: 48, LCDR2 having the amino acid sequence of SEQ ID NO: 49, and LCDR3 having the amino acid sequence of SEQ ID NO: 50.

In one embodiment, the antibody or antigen-binding fragment thereof of the present invention comprises a heavy chain variable domain having the amino acid sequence of SEQ ID NO: 14, and a light chain variable domain having the amino acid sequence of SEQ ID NO: 9.

In one embodiment, the antibody or antigen-binding fragment thereof of the present invention comprises a heavy chain variable domain having the amino acid sequence of SEQ ID NO: 22, and a light chain variable domain having the amino acid sequence of SEQ ID NO: 9.

In one embodiment, the antibody or antigen-binding fragment thereof of the present invention comprises a heavy chain variable domain having the amino acid sequence of SEQ ID NO: 30, and a light chain variable domain having the amino acid sequence of SEQ ID NO: 9.

In one embodiment, the antibody or antigen-binding fragment thereof of the present invention comprises a heavy chain variable domain having the amino acid sequence of SEQ ID NO: 38 and a light chain variable domain having the amino acid sequence of SEQ ID NO: 46.

In the present invention, SEQ ID NOS: 3, 4, 5, 16, 17, 18, 24, 25, 26, 32, 33, 34, 40, 41, and 42 are CDRs defined by the Kabat numbering system based on sequence comparison.

In the present invention, SEQ ID NOS: 6, 7, 8, 19, 20, 21, 27, 28, 29, 35, 36, 37, 43, 44, and 45 are CDRs defined by the Chothia numbering system based on sequence comparison.

Meanwhile, SEQ ID NOS: 11 to 13, and 54 to 56 may be CDRs defined by the Kabat or Chothia numbering system.

In the present invention, SEQ ID NOS: 51 to 71 are CDRs defined by the IMGT numbering system based on sequence comparison.

The TM4SF5-binding antibody of the present invention is an antibody capable of inhibiting the activity of the TM4SF5 protein by binding to the TM4SF5 protein, and exhibits the characteristic of binding to the TM4SF5 protein with high affinity.

As used herein, the term "transmembrane 4 L6 family member 5 (TM4SF5)" is a type belonging to the transmembrane 4 superfamily (TM4SF), which is a group of membrane receptors that pass through the cell membrane four times, and refers to a protein that mediates signaling pathways involved in cell development, activation, growth, and regulation of migration. The TM4SF5 protein has four domains that pass through the cell membrane, two loop structures present extracellularly, and one loop structure and two tails present in the cytoplasm. Among the two loop structures present extracellularly, the length of the second external loop, or extracellular loop 2 (EC2), is longer than the first external loop, or extracellular loop 1 (EC1), and key amino acid residues involved in the interaction with other molecules are present in EC2.

The type of the TM4SF5 protein is not particularly limited, but may preferably be a human TM4SF5 protein. Additionally, the TM4SF5 protein includes both native and mutant TM4SF5 proteins, but is not limited thereto. The native TM4SF5 protein generally refers to a polypeptide comprising the amino acid sequence of the native TM4SF5 protein, and the amino acid sequence of the native TM4SF5 protein generally refers to the amino acid sequence found in naturally occurring TM4SF5 protein. The information on TM4SF5 can be obtained from a known database (*e.g*., GenBank of the National Institutes of Health), and for example, it may be the information on the TM4SF5 protein having an Accession Number of NP_003954 (Gene ID: 9032), but is not limited thereto.

Meanwhile, the expression of "having the amino acid sequence of" a specific sequence number may be construed as comprising the amino acid sequence, and in some embodiments, it may be modified to consisting essentially of or consisting of the amino acid sequence.

As an example of the method for preparing the antibody of the present invention, the method may be performed by preparing a hybridoma using B lymphocytes obtained from an immunized animal (Koehler and Milstein, 1976, Nature, 256:495), or may be performed by using a phage display technology, but is not limited thereto.

An antibody library using a phage display technology is a method of obtaining a gene of an antibody directly from B lymphocytes without preparing a hybridoma and expressing an antibody on the surface of a phage with the gene. Many of the existing difficulties associated with the production of monoclonal antibodies via B-cell immortalization can be overcome by using the phage display method. In general, a phage display method consists of: 1) inserting an oligonucleotide with a random sequence into the gene portion corresponding to the N-terminus of phage coat protein pIII (or plV); 2) expressing a fusion protein having a part of a native coat protein and a polypeptide encoded by the oligonucleotide having the random sequence; 3) treating a receptor material that can bind to the polypeptide encoded by the oligonucleotide; 4) eluting peptide-phage particles bound to the receptors at a low pH condition or using a binding-competitive molecule; 5) amplifying the eluted phage in a host cell by panning; 6) repeating the above steps to obtain a desired amount of phage; and 7) determining the sequence of a peptide with activity from the DNA sequences of the phage clones selected by panning.

For example, the method for preparing the monoclonal antibody of the present invention may be performed using a phage display technology. Those skilled in the art can easily perform the preparation of an antibody of the present invention with reference to known phage display technology, for example, the methods disclosed in literature references including Barbas et al. (METHODS: A Companion to Methods in Enzymology 2:119, 1991 and J. Virol. 2001 Jul; 75(14):6692-9) and Winter et al. (Ann. Rev. Immunol. 12:433, 1994). For example, known yeast display technology may also be used.

The polynucleotide encoding the monoclonal antibody or display clone of the present invention can be easily isolated and analyzed for its sequence using conventional procedures. For example, oligonucleotide primers designed to specifically amplify the heavy chain and light chain coding regions from hybridoma or phage template DNA may be used. Once the polynucleotide is isolated, it can be inserted into an expression vector, which can then be transformed into a suitable host cell to obtain, from the transformed host cell (*i.e*., transformants), a desired monoclonal antibody.

Accordingly, the method for preparing an antibody or antigen-binding fragment thereof of the present invention may comprise amplifying a polynucleotide encoding the antibody or antigen-binding fragment thereof in an expression vector comprising the polynucleotide encoding the antibody or antigen-binding fragment thereof, but is not limited thereto.

The expression vector comprising a polynucleotide encoding an antibody or antigen-binding fragment thereof of the present invention is not particularly limited, but may be a vector capable of replicating and/or expressing the polynucleotide in eukaryotic or prokaryotic cells, including mammalian cells (*e.g*., human, monkey, rabbit, rat, hamster, and mouse cells), plant cells, yeast cells, insect cells, and bacterial cells (*e.g., E. coli*)*.* The expression vector may be a vector operably linked to a suitable promoter such that the polynucleotide can be expressed in a host cell, and that comprises at least one selective marker. For example, it may be in a form in which the polynucleotide is introduced into a phage, plasmid, cosmid, mini-chromosome, virus, or retroviral vector.

The expression vector comprising a polynucleotide encoding an antibody or antigen-binding fragment thereof may be an expression vector comprising a polynucleotide encoding either the heavy chain or light chain of the antibody, or an expression vector comprising both the polynucleotide encoding the heavy chain and the polynucleotide encoding the light chain.

In the present invention, the transformant into which the expression vector has been introduced is not particularly limited thereto, but may be bacterial cells such as *Escherichia coli, Streptomyces,* or *Salmonella typhimurium;* yeast cells; fungal cells such as *Pichia pastoris;* insect cells such as *Drosophila* or *Spodoptera* Sf9 cells; animal cells such as Chinese hamster ovary (CHO) cells, SP2/0 (mouse myeloma), human lymphoblastoid, COS, NSO (mouse myeloma), 293T, B16 melanoma cells, HT-1080, baby hamster kidney (BHK) cells, human embryonic kidney (HEK) cells, or PER.C6 (human retinal cells); or plant cells, into which the expression vector has been introduced and transformed.

As used herein, the term "introduction" refers to a method of delivering the vector comprising a polynucleotide encoding an antibody or antigen-binding fragment thereof into a host cell. Such an introduction may be performed by various methods known in the art, including calcium phosphate-DNA co-precipitation, DEAE-dextran-mediated transfection, polybrene-mediated transfection, electroporation, microinjection, liposome fusion, lipofectamine and protoplast fusion methods, *etc.* Additionally, transfection refers to the delivery of a target material into a cell by means of infection using viral particles. Further, the vector may be introduced into a host cell by gene bombardment, *etc.* In the present invention, the term "introduction" may be used interchangeably with the term "transfection".

The pharmaceutical composition of the present invention may further comprise a pharmaceutically acceptable carrier in addition to the antibody or antigen-binding fragment thereof of the present invention, and the carrier may include a non-naturally occurring carrier.

As used herein, the term "pharmaceutically acceptable carrier" refers to a carrier or diluent that does not cause irritation to an organism and does not inhibit the biological activities or properties of the compound to be administered. Examples of the pharmaceutically acceptable carrier permitted for compositions formulated as liquid solutions may include sterile and biocompatible carriers, including saline solution, sterile water, Ringer's solution, buffered saline solution, albumin injection solution, dextrose solution, maltodextrin solution, glycerol, ethanol, and a mixture of one or more of these components thereof, and upon necessity, may also include other conventional additives such as antioxidants, buffers, and bacteriostatic agents. Additionally, by further adding diluents, dispersing agents, surfactants, binders, and lubricants, the composition may be formulated into injectable formulations such as aqueous solutions, suspensions, or emulsions, or into pills, capsules, granules, or tablets.

The pharmaceutical composition may have any one formulation selected from the group consisting of tablets, pills, powders, granules, capsules, suspensions, liquid solutions for internal use, emulsions, syrups, sterilized aqueous solutions, non-aqueous solvents, suspensions, emulsions, lyophilized formulations, and suppositories, and may be in the form of various oral or parenteral formulations. The pharmaceutical composition is formulated using commonly used diluents or excipients, such as fillers, extenders, binders, wetting agents, disintegrants, or surfactants. Solid formulations for oral administration include tablets, pills, powders, granules, and capsules. These solid formulations are prepared by mixing at least one compound with at least one excipient, for example, starch, calcium carbonate, sucrose or lactose, gelatin, *etc*. In addition to simple excipients, lubricants such as magnesium stearate and talc may also be used. Liquid formulations for oral administration include suspensions, solutions for internal use, emulsions, syrups, *etc.* In addition to water and liquid paraffin, which are commonly used simple diluents, various excipients (*e.g*., wetting agents, sweetening agents, fragrances, and preservatives) may also be comprised. Formulations for parenteral administration include sterilized aqueous solutions, non-aqueous solvents, suspending agents, emulsions, lyophilized agents, and suppositories. As non-aqueous solvents and suspending agents, propylene glycol, polyethylene glycol, vegetable oils (*e.g*., olive oil), injectable esters (*e.g*., ethyl oleate), *etc*. may be used. As bases for suppositories, Witepsol, Macrogol, Tween 61, cacao butter, laurin butter, glycerinated gelatin, *etc.* may be used.

Additionally, the pharmaceutical composition of the present invention may be administered in a pharmaceutically effective amount.

As used herein, the term "pharmaceutically effective amount" refers to an amount sufficient to treat a disease at a reasonable benefit/risk ratio applicable to medical treatment, and the effective dosage level may be determined depending on factors including the type and severity of the subject, age, sex, type of disease, activity of the drug, sensitivity to the drug, time of administration, route of administration and excretion rate, duration of treatment, and drugs used concurrently, as well as other factors well known in the medical field. The composition of the present invention may be administered as an individual therapeutic agent or in combination with other therapeutic agents, and may be administered sequentially or simultaneously with conventional therapeutic agents. The composition may be administered in a single or multiple dosage form. It is important to administer the composition in a minimum amount capable of exhibiting the maximum effect without causing side effects in view of all the above-described factors, and the dose can easily be determined by those skilled in the art.

Additionally, the pharmaceutical composition may be administered orally or parenterally (*e.g*., intravenous, subcutaneous, intraperitoneal, or topical application) depending on the desired method. For example, the pharmaceutical composition may be administered parenterally.

The pharmaceutical composition of the present invention may be administered as an individual therapeutic agent or together with other active ingredients exhibiting preventive or therapeutic effects on a TM4SF5-related disease. When the pharmaceutical composition of the present invention is administered together with other active ingredients, this administration may be called "combination administration", "co-administration", or "complex administration". In particular, the TM4SF5-binding antibody or antigen-binding fragment thereof of the present invention and other active ingredients may be administered as a mixture or in separate forms.

In the present invention, the terms "co-administration", "co-administered", and "co-administering" should be understood as referring to simultaneous, individual, sequential, or reverse order of administration, in any order. That is, co-administration is not merely limited to simultaneous administration, but may be a form of administration in which each material can exhibit a level equal to or higher than its original function by acting together on the subject. Therefore, when the term "co-administration" is used herein, the order of administration is not particularly limited when the administration is performed sequentially, in a reverse order, or individually, and the interval of administration of the second component can be determined so as to ensure that the beneficial effects of the co-administration are not lost.

For example, the pharmaceutical composition of the present invention may be administered together with a material selected from the group consisting of 5-fluorouracil (5-FU), oxaliplatin, doxorubicin, sorafenib, and cetuximab.

In an embodiment of the present invention, doxorubicin or sorafenib was administered in combination with the antibody of the present invention, and excellent effects resulting therefrom were confirmed.

A TM4SF5-related disease of the present invention is a disease caused by the overexpression of TM4SF5. For example, the TM4SF5-related disease may be a disease selected from fatty liver, liver fibrosis, liver cancer, stomach cancer, colorectal cancer, rectal cancer, oral cancer, pharynx cancer, larynx cancer, lung cancer, colon cancer, breast cancer, cervical cancer, endometrial cancer, ovarian cancer, prostate cancer, testicular cancer, bladder cancer, kidney cancer, pancreatic cancer, bone cancer, connective tissue cancer, skin cancer, brain cancer, thyroid cancer, leukemia, Hodgkin's disease, soft tissue sarcoma, lymphoma, and multiple myeloma hematologic cancer.

For example, it is known that, in cancer, the TM4SF5 protein induces epithelial-mesenchymal transition (EMT), which is involved in the development, invasion, or metastasis of cancer, and ultimately causes the loss of contact inhibition of cells, thereby leading to multilayer growth (Lee SA et al., J Clin Invest 2008, 118(4):1354-66). Additionally, the TM4SF5 protein expressed in a cancer cell intracellularly interacts with integrin α5, thereby activating the signaling process of FAK/c-Src/STAT3 and causing the expression and secretion of vascular endothelial growth factor (VEGF), an important factor in angiogenesis, potentially causing the angiogenesis of vascular endothelial cells (Choi S et al., Blood 2009 113(8):1845-55). Accordingly, any material that inhibits the function of TM4SF5 can exhibit an anticancer effect.

In an embodiment of the present invention, the effect of an antibody that specifically binds to TM4SF5 protein on the improvement of symptoms of colon cancer, liver cancer, prostate cancer, stomach cancer, *etc.* was confirmed; accordingly, the antibody or antigen-binding fragment thereof of the present invention can be used for the prevention or treatment of diseases caused by overexpression of TM4SF5 protein.

As used herein, the term "prevention" may refer to any activity that inhibits or delays the onset of a disease by administering the composition of the present invention. Additionally, as used herein, the term "treatment" may refer to any action that improves or beneficially changes the symptoms of a disease by administering the composition of the present invention.

Overexpression of TM4SF5 protein is observed in various cells and is associated with the onset of various diseases. Thus, the antibody or antigen-binding fragment thereof of the present invention, which binds to TM4SF5 protein with high specificity and affinity, can be usefully employed for diagnosing diseases related to TM4SF5 protein expression or the expression level thereof.

In the method for providing information for diagnosis, TM4SF5 protein can be detected by reacting the TM4SF5-specific antibody of the present invention with a biological sample isolated from a subject suspected of having a disease, and detecting the formation of an antigen-antibody complex, and through this method, information for cancer diagnosis can be provided.

Specifically, the method may be a method for providing information for diagnosis of cancer or a method for diagnosing cancer, which includes: (a) treating the antibody to an isolated biological sample of a subject suspected of having a TM4SF5-related disease and detecting TM4SF5 protein through an antigen-antibody reaction; and (b) comparing the level of TM4SF5 protein detected in (a) with that of the control group, so that if the TM4SF5 protein level is higher than that of the control group, the subject is determined as a patient having the TM4SF5-related disease.

As used herein, the term "biological sample" may include a tissue, a cell, whole blood, serum, plasma, a tissue autopsy sample (*e.g*., brain, skin, lymph node, or spinal cord), a cell culture supernatant, a ruptured eukaryotic cell, a bacterial expression system, *etc.,* but is not limited thereto. The presence of the TM4SF5 protein or the presence of cancer can be confirmed by reacting these biological samples with the antibody of the present invention in a manipulated or unmanipulated state.

As used herein, the term "antigen-antibody complex" refers to a conjugate of a TM4SF5 protein antigen in a sample and the antibody of the present invention capable of recognizing the antigen. The formation of such an antigen-antibody complex can be detected by any method, such as a colorimetric method, an electrochemical method, a fluorimetric method, a luminometric method, a particle counting method, visual assessment, and a scintillation counting method, but the method is not limited thereto, and various methods may be used and applied.

Various labels may be used to detect the antigen-antibody complex. Specific examples include enzymes, fluorescent materials, ligands, luminescent materials, microparticles, and radioactive isotopes, but are not limited thereto. Examples of enzymes that may be used as the detection label include acetylcholinesterase, alkaline phosphatase, β-D-galactosidase, horseradish peroxidase, β-lactamase, *etc.* Examples of the fluorescent materials include fluorescein, Eu³⁺, Eu³⁺ chelate, cryptate, *etc.* Examples of the ligands include biotin derivatives, *etc.* Examples of the luminescent materials include acridinium ester, isoluminol derivatives, *etc.* Examples of the microparticles include colloidal gold, colored latex, *etc.* Examples of the radioactive isotopes include ⁵⁷Co, ³H, ¹²⁵I, ¹²⁵I-Bonton Hunter reagents, *etc.*

For example, the antigen-antibody complex may be detected by an ELISA method. Examples of the ELISA method include various ELISA methods, such as a direct ELISA which uses a labeled antibody capable of recognizing the antigen attached to a solid support; an indirect ELISA which uses a labeled secondary antibody capable of recognizing a capture antibody in an antibody complex capable of recognizing an antigen attached to a solid support; a direct sandwich ELISA which uses another labeled antibody capable of recognizing an antigen in an antigen-antibody complex attached to a solid support; and an indirect sandwich ELISA, which involves reacting with another antibody capable of recognizing an antigen in an antigen-antibody complex attached to a solid support and then using the labeled secondary antibody capable of recognizing the antibody.

The antibody may have a detection label. When the antibody does not have a detection label, the antibody can be captured, and it can be confirmed by treating the antibody with another antibody having a detection label.

### [Detailed Description Of The Invention]

Hereinafter, the present invention will be described in more detail with reference to the following Examples and Experimental Examples. However, these Examples and Experimental Examples are for illustrative purposes only, and the scope of the present invention is not limited by these Examples and Experimental Examples.

### Example 1 Construction of humanized Ab27 antibody and selection of huAb27-9

### Example 1-1: Design of humanized antibody and selection of huAb27-9

To humanize the Ab27 antibody, a TM4SF5-binding antibody previously identified through prior studies, human framework donor was selected by performing both a fixed approach, which employs a specific framework, and a best-fit approach based on homology matching.

As a fixed framework, the Herceptin (Genentech) backbone (Human Heavy Chain Subgroup III, Human Kappa Light Chain Subgroup I), known for its excellence in stability and immunogenicity, was used to design huAb27-1, huAb27-2, and huAb27-3 by considering CDR-grafting and backmutation. Although the FR backbone of Herceptin is known to be structurally stable and to have low immunogenicity, when Ab27 was grafted onto this FR backbone, antigen-binding affinity was observed to be less than 30% of that of the chimeric antibody. Therefore, the strategy was switched to a best-fit approach in which the FR backbone is selected based on homology.

Using the best-fit approach, the frameworks IGHV1, IGHV5, IGHV7 (Human Heavy Chain Subgroup I), IGKV2 (Human Kappa Light Chain Subgroup II), IGKV3 (Human Kappa Light Chain Subgroup III), and IGKV4 (Human Kappa Light Chain Subgroup IV) were used to design huAb27-10 through huAb27-111 by considering CDR-grafting, back-mutation, structure-stabilizing mutations, and the FR backbone.

As the primary selection criterion for the humanized antibodies obtained by transient expression, binding affinity was measured using the following method. Using Biacore T200 (GE Healthcare), anti-human IgG (Fc) antibodies were immobilized on the CM5 chip surface to capture the humanized antibodies, followed by injection of hEC2-GST-His protein as the analyte to measure the binding affinity. First, an immobilization step was performed according to the manufacturer's protocol for the Human Ab Capture Kit (GE Healthcare, two22-0648-88), and HBS-EP (GE Healthcare, BR-10-0669) was used as the sample and system buffer. The Ab27 antibody was diluted from 200 nM by two-fold serial dilution, and the binding affinity was measured at a flow rate of 30 µL/min with 2 minutes of association and 5 minutes of dissociation. In each cycle, regeneration was performed by injecting 3 M MgCl₂ at 30 µL/min for 30 seconds. After the completion of the kinetics measurements, K_{D} values were calculated using the 1:1 binding model in the BIAevaluation software version 1.0.

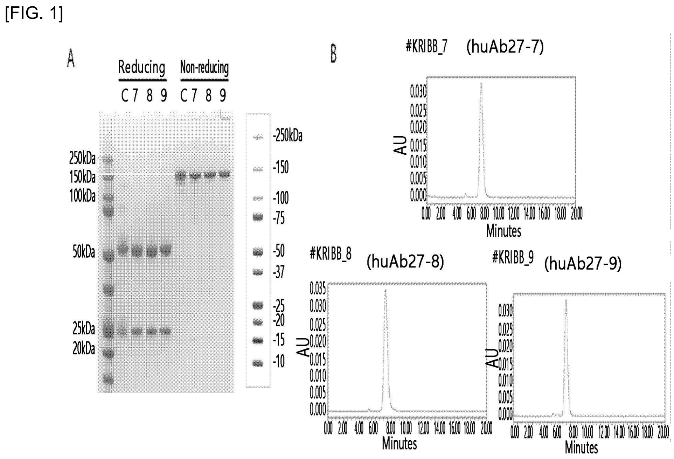

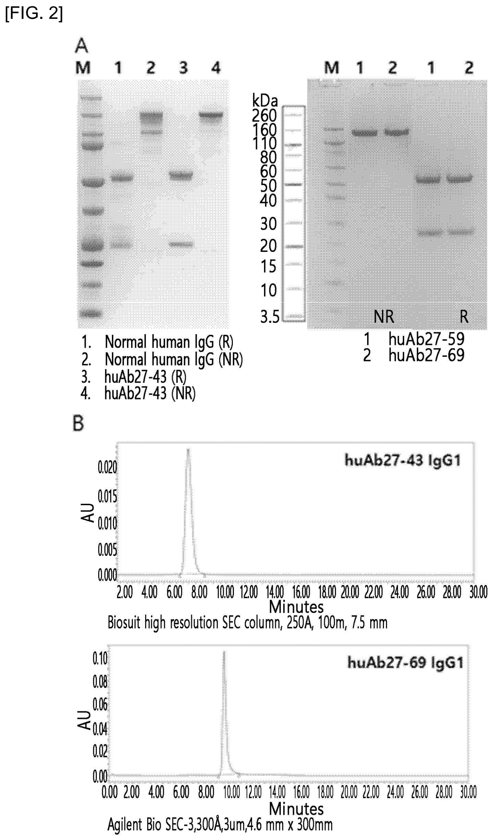

Among the constructed antibodies, huAb27-8, huAb27-9, huAb27-43, and huAb27-69 were produced using the same vector and expression system as the chimeric antibody, and their physicochemical properties (FIGS. 1 and 2) and binding affinity were confirmed.

scFv-Fc reformatting was performed as follows:
The expression vector was constructed by amplifying huAb27-8 and huAb27-9 using PCR and inserting the same into the FUSE-hlgG1-Fc2 (Invivogen, pfuse-hg1fc2) vector using the In-Fusion^{®} HD Cloning Kit (Clontech, 638909). This method performs cloning in a homologous overlap manner by linearizing only the vector to be ligated, without digesting the amplified insert DNA with restriction enzymes, and using identical sequences of approximately 15-18 bp at both termini. Using the IL-2 signal peptide sequence present in the vector as the signal sequence, pFUSE-huAb27-8 scFv-Fc and pFUSE-huAb27-9 scFv-Fc were constructed. The VH, VL, and Fc regions of huAb27-43 and huAb27-69 were PCR-amplified and cloned into the pFUSE vector using the EcoRI/BamHI restriction enzymes, thereby constructing pFUSE-huAb27-43 scFv-Fc and pFUSE-huAb27-69 scFv-Fc, respectively.

Freestyle^{™} 293-F (Invitrogen, R79007) cells were transfected with each scFv-Fc expression vector plasmid DNA at a 1:1 ratio using PEI. One day prior to transfection, HEK293F cells were cultured at a density of 1 × 10⁶ cells/mL in 300 mL culture. On the following day, transfection was performed using polyethylenimine (PEI, Polyscience #23966). The two types of constructs were mixed in 30 mL of Freestyle 293 Expression Medium (Gibco, #12338-018) at a Construct:PEI ratio of 1:2. After incubation at room temperature for 20 minutes, the mixtures were added to the pre-cultured cells. The cells were then cultured in a shaking CO₂ incubator at 37°C, 125 rpm, and 8% CO₂ for 5 days. After the culture was centrifuged, the supernatant was filtered (Corning #431174), and subsequently subjected to purification.

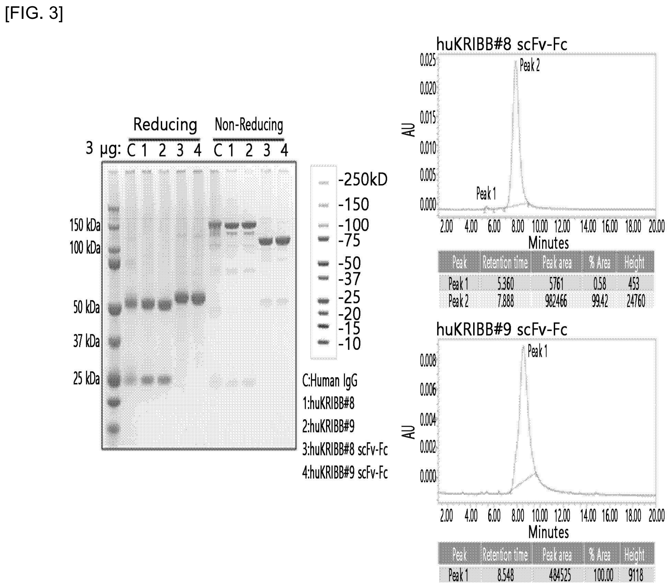

Antibody proteins were purified using a HiTrap MabSelect SuRe (Protein A affinity chromatography) column. The purified antibodies were analyzed by using SDS-PAGE under reducing and non-reducing conditions and by SE-HPLC (Waters HPLC, Alliance 2695, UV/Vis Detector 2489) to confirm purity and mobility (FIG. 3).

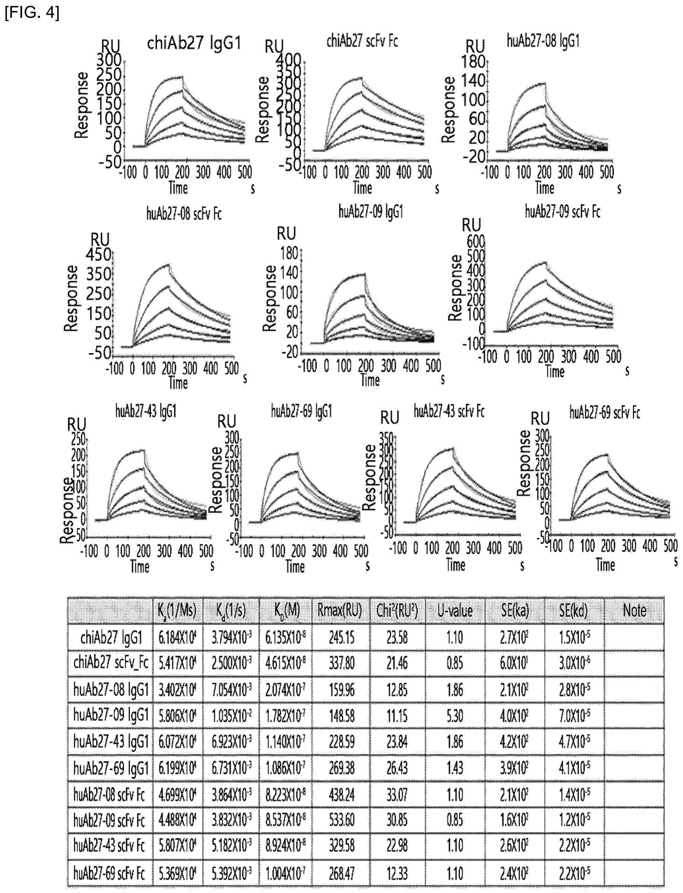

Using the same method, huAb27-8, huAb27-9, huAb27-43, and huAb27-69 were constructed in both IgG1 and scFv-Fc formats, expressed, and purified, and their binding affinity was evaluated (FIG. 4).

### Example 1-2: Large-scale production and additional validation

Large-scale production of the antibodies was performed for use in *in vitro* and *in vivo* efficacy tests.

### Transformation of expression vector

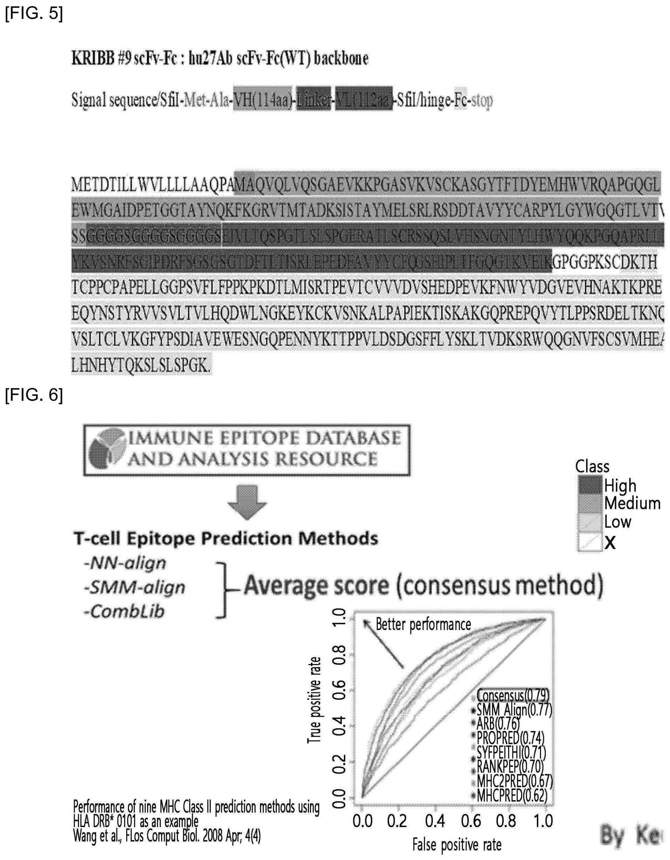

The huAb27-9 scFv-Fc sequence cloned into the existing pFUSE-hlgG-Fc2 vector was introduced into the backbone of Ab27 vector to perform subcloning. Restriction enzyme Sfil was used, and as the signal sequence, the original signal sequence present in the Ab27 vector was used as is to complete cloning. The amino acid sequence of huAb27-9 scFv-Fc antibody is shown in FIG. 5.

Ab27 scFv-Fc was transiently expressed in 1 L of ExpiCHO cell line, and huAb27-9 scFv-Fc was transiently expressed in 500 mL of the same, followed by purification. Purification was carried out using AKTAprime, and the entire culture medium was divided into 50 mL portions and loaded onto the column for binding. The purification conditions are shown in Table 1.

**[Table 1]**

| Purificati on method | Column | Elution Buffer | Neutralizati on |
|---|---|---|---|
| Affinity Chromatography | HiTrap | 0.1M Citrate Buffer + 5% Sucrose (pH 3.0) | 1M Tris (pH 9.0) |
| | MabSelectSuRe(GE Healthcare) | | |

After purification, buffer exchange and concentration were carried out using 1× PBS. During the concentration process, slight precipitation was observed. The final concentrations of the recovered antibodies are shown in Table 2.

**[Table 2]**

| Equipme nt | Antibody | Final concentration | Total recovery |
|---|---|---|---|
| Nanodro p | Ab27 (1st) | 0.85 mg/mL | 68.8 mg |
| | Ab27 (2nd) | 1.2 mg/mL | 78 mg |
| | huAb27-9 | 1.5 mg/mL | 100 mg |

### Immunogenicity assessment

*In silico* immunogenicity prediction based on IEDB was used for HLA types corresponding to MHC class I and MHC class II (FIG. 6). Based on *in vitro* IC₅₀ experimental values for nine amino acid sequences, the Consensus method, which is a combination of *in silico* approaches whose prediction performance has been validated, was used for MHC class I (ANN, SMM, CombLib, NetMHCpan) and MHC class II (NN-align, SMM-align, CombLib, NetMHCllpan). For the immunogenicity analysis of the given amino acid sequences, the immunogenicity prediction results were obtained by using an internally established web-based analysis system, which only requires the amino acid sequence information to be entered to quickly and readily select the HLA types for MHC class I and MHC class II. Twenty-six HLA types frequently observed in many individuals were analyzed, and the results were visualized in red, orange, and yellow from sequences with high epitope potential to those with low potential.

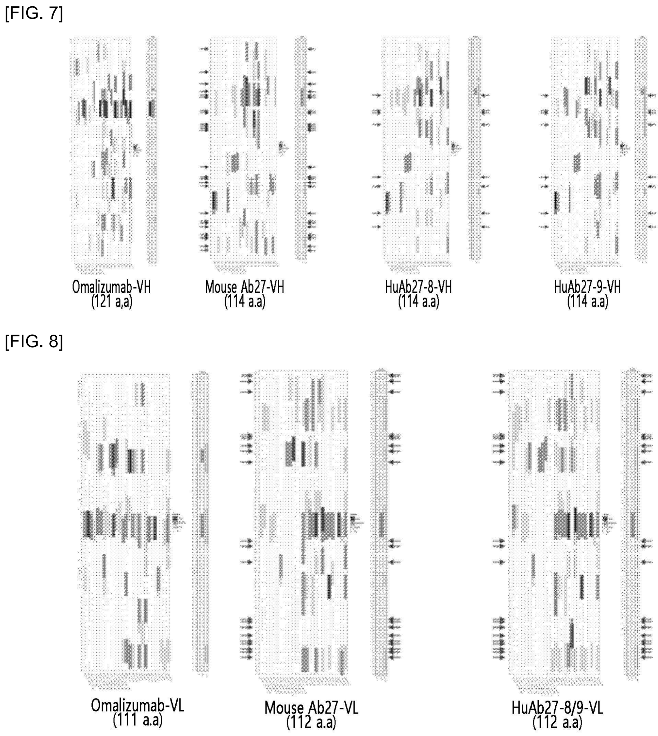

Immunogenicity of the VH region was predicted in the order of huAb27-8 (low) < huAb27-9 (medium) < Mouse Ab27 (high) (FIG. 7). Both huAb27-8 and huAb27-9 were predicted to have lower immunogenicity than Omalizumab, a commercially available antibody drug known to have 0% immunogenicity. In addition, the immunogenicity of the VL region was predicted to be similar for both mouse and human (FIG. 8). huAb27-8 and huAb27-9 were predicted to have immunogenicity comparable to that of Omalizumab, a commercially available antibody drug known to have 0% immunogenicity.

### Affinity assessment

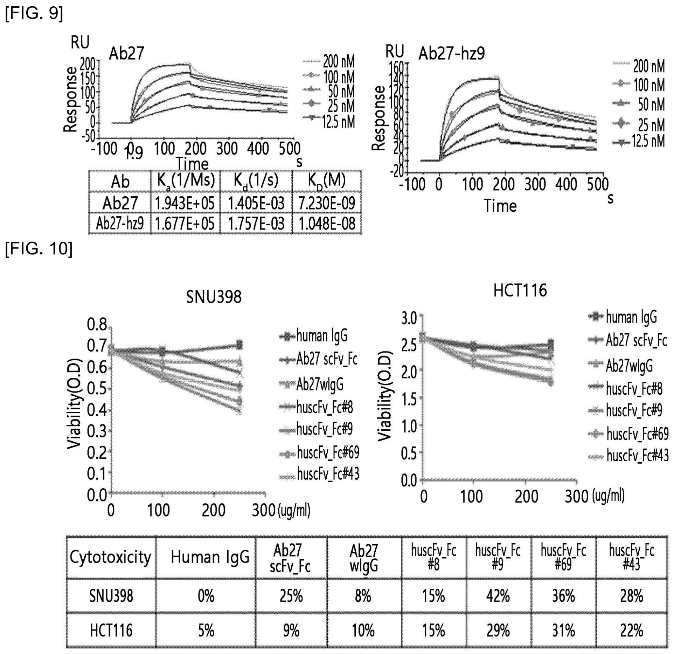

SPR analysis of Ab27 scFv-Fc and humanized Ab27-9 scFv-Fc showed K_{D} values of 10.48 nM and 7.23 nM, respectively, indicating approximately a 1.4-fold difference in affinity (FIG. 9).

### Cytotoxicity assay on cancer cell lines

When the humanized antibodies produced above were treated at 100 µg/mL and 250 µg/mL to assess cytotoxicity, clear cell growth inhibition was observed at treatment with 250 µg/mL. In SNU398 cells, growth inhibition was observed in the order of #9 > #69 > #43 > Ab27 scFv_Fc > #8 > Ab27 wIgG, and in HCT116 cells, growth inhibition was observed in the order #69 > #9 > #43 > #8 > Ab27 wIgG > Ab27 scFv-Fc (FIG. 10).

Overall, the humanized antibody #9 scFv-Fc showed superior affinity and cancer cell growth inhibition compared to Ab27 scFv-Fc; therefore, the humanized antibody #9 (huAb27-9) was selected as the humanized antibody for subsequent optimization.

The sequence of the humanized antibody #9 (huAb27-9) is shown in SEQ ID NOS: 1 to 13. Specifically, the VH sequence is shown in SEQ ID NO: 1, the VL sequence in SEQ ID NO: 9, the HCDR sequences defined by Kabat numbering are shown in SEQ ID NOS: 3 to 5, and the HCDR sequences defined by Chothia numbering in SEQ ID NOS: 6 to 8. The LCDR sequences defined by Kabat and Chothia numbering were identical, and they are shown in SEQ ID NOS: 11 to 13. The full-length heavy chain and light chain sequences are shown in SEQ ID NOS: 2 and 10, respectively.

### Example 2: Affinity optimization of the huAb27-9 antibody

In the affinity optimization of the huAb27-9 antibody, two approaches, untargeted mutagenesis and targeted mutagenesis, were used.

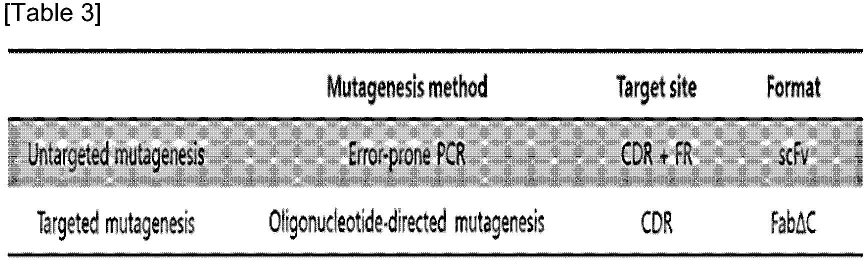

### Example 2-1: Screening of untargeted library and identification of E01 antibody

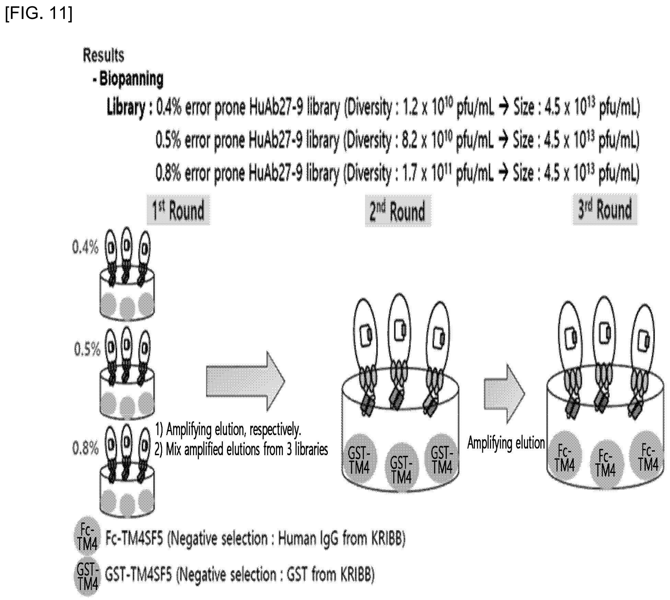

To perform affinity optimization using untargeted mutagenesis, mutations were introduced into both the CDRs and FRs in the scFv format. A library was generated using the error-prone PCR method at error rates of 0.4%, 0.5%, and 0.8%, and clones were selected through a panning process (FIG. 11).

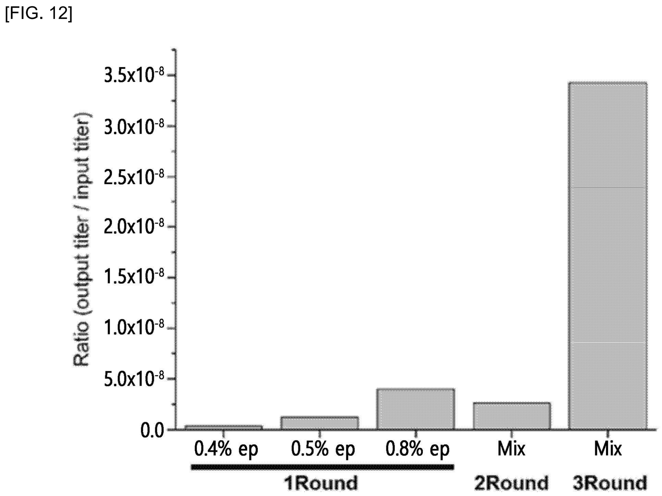

Through the panning process described above, it was confirmed that enrichment occurred in the third round of panning (FIG. 12).

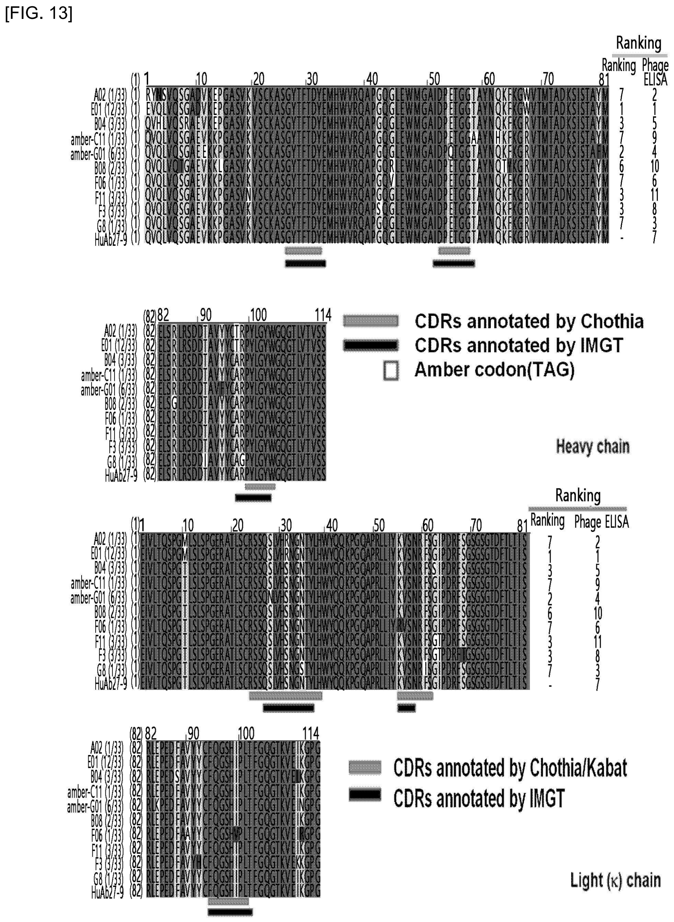

A total of 33 clones were selected using ELISA and subjected to sequence analysis, the results of which are shown below. A certain degree of correlation was observed in that clones repeatedly enriched during the panning process exhibited high binding in phage ELISA (FIG. 13).

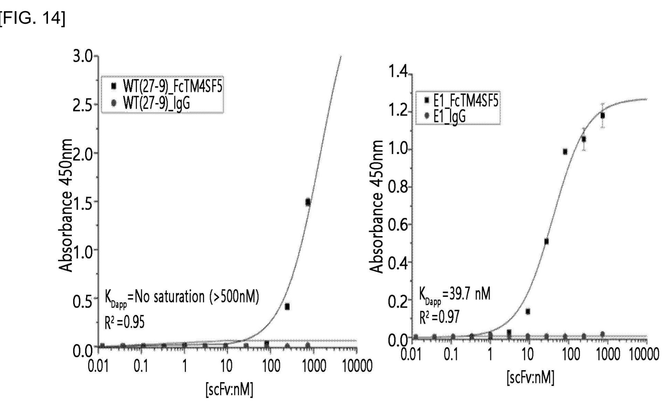

The above clones were expressed in *E. coli* in the scFv format and the purified proteins were used to measure binding affinity using ELISA. As a result, the E1 clone exhibited improved binding affinity. In the concentration range of 0.01 nM to 800 nM, six clones including WT did not reach saturation, and thus, apparent K_{D} values could not be determined. However, the E1 clone exhibited a saturation pattern within the same concentration range, and its K_{D} value was predicted to be 39.7 nM, suggesting a more than 10-fold improvement in binding affinity (FIG. 14).

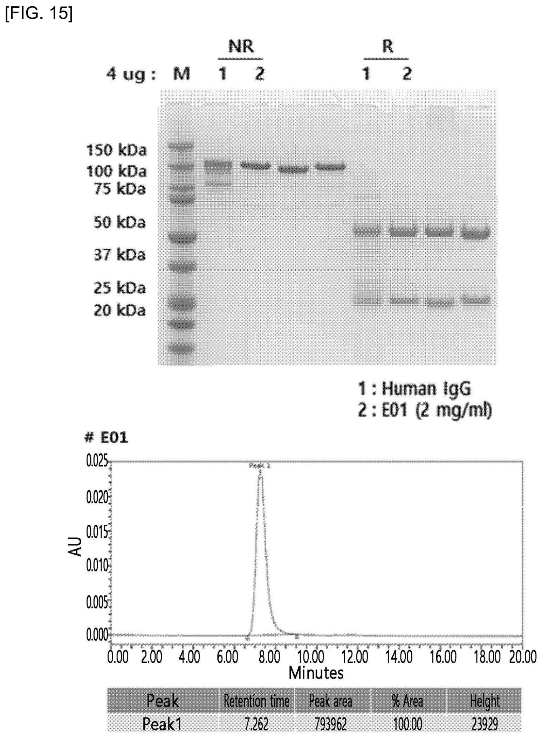

The selected antibody was converted to the IgG1 format and transiently expressed in Expi293 cells, followed by purification using a MabSelect SuRe column. After purification, SDS-PAGE and SE-HPLC analyses were performed to confirm antibody size and physicochemical properties (FIG. 15).

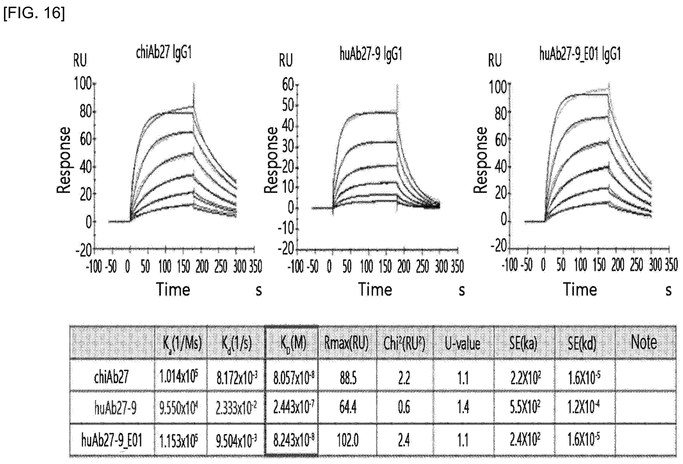

Using the purified protein in IgG1 format, binding affinity was measured by antigen direct immobilization, in which the TM4SF5-Fc antigen protein was immobilized on the chip surface (FIG. 16). As a result, the antigen-binding affinity improved three-fold compared to that of the parent antibody huAb27-9, and was observed to be comparable to that of the chimeric antibody.

### Example 2-2: Screening of targeted library

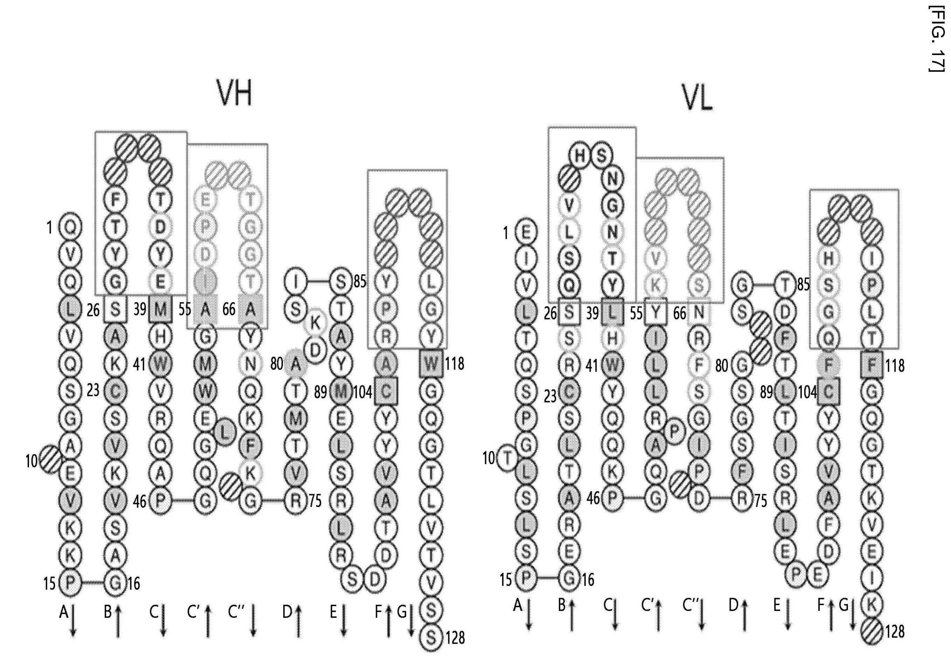

To perform affinity optimization using targeted mutagenesis, a randomized library of the CDR regions, including hotspot positions, was constructed in the FabΔC format. The target regions in the CDRs were selected based on the portions exposed as loops within the CDR regions defined according to the Kabat/IMGT criteria (FIG. 17).

A library with a functional diversity of more than 80% at the 10⁸ diversity level, exhibiting a diversity 30 to 70 times higher than the theoretical diversity, was generated.

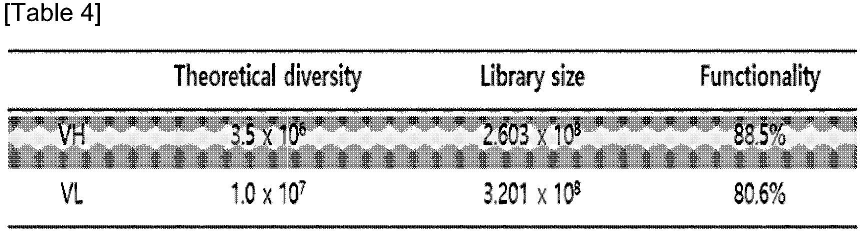

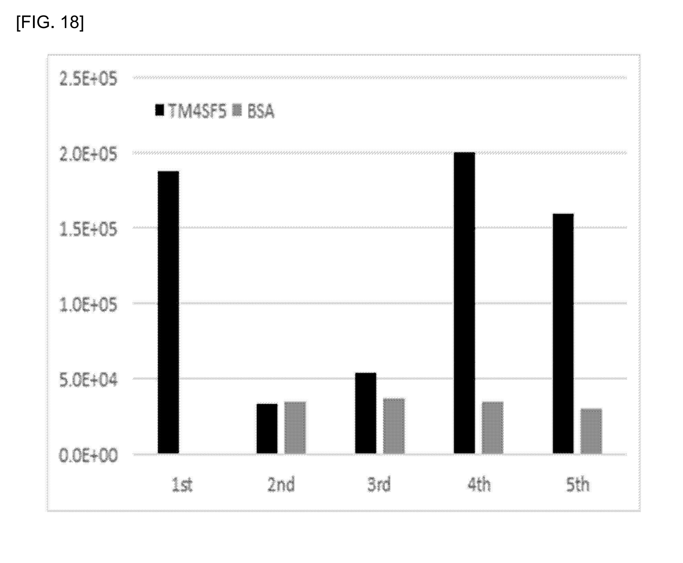

The CDR-randomized library prepared as above was screened through a panning process using phage display technology. Enrichment was observed starting from the fourth round of panning (FIG. 18).

**[Table 5]**

| Panning results of CDR-randomized library | | | | |
|---|---|---|---|---|
| | TM 4SF5 | BS A | Rat io | Me thod |
| 1st | 1.9E+05 | | | bead |
| 2nd | 3.4E+04 | 3.5E+04 | 1.0 | tube |
| 3rd | 5.4E+04 | 3.7E+04 | 1.5 | bead |
| 4th | 2.0E+05 | 3.5E+04 | 5.7 | bead |
| 5th | 1.6E+05 | 3.1E+04 | 5.2 | tube |

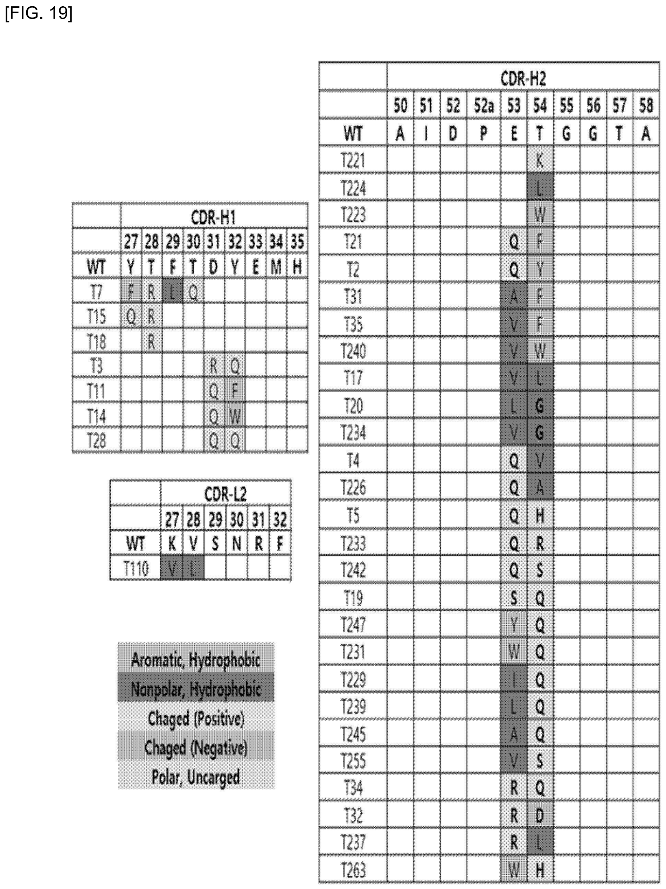

Sequence analysis of the selected clones revealed that mutations were mainly concentrated in CDR-H1 and CDR-H2, and mutations were also observed in CDR-L2 (FIG. 19). However, clones derived from CDR-L2 did not exhibit significantly increased binding affinity compared to that of WT, and therefore, a combinational library of CDR-H1 and CDR-H2, excluding the clones derived from CDR-L2 was constructed and screened.

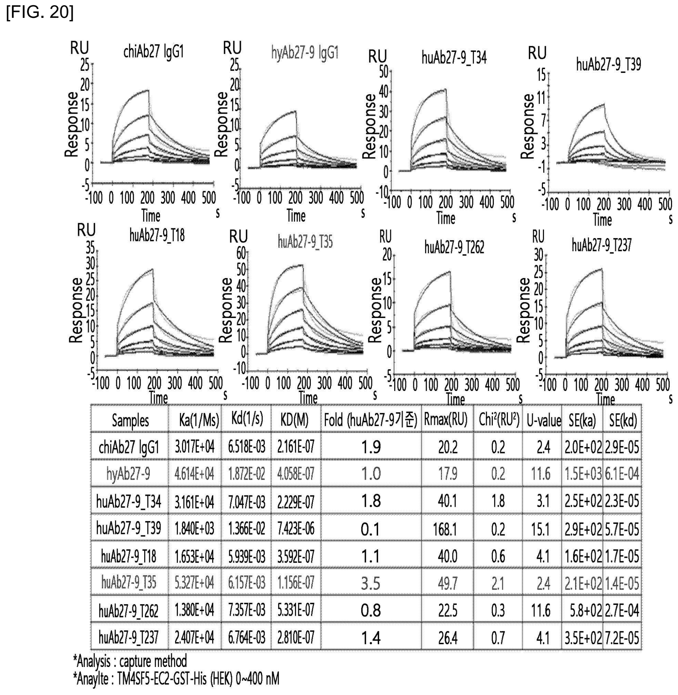

The selected antibodies were converted to the IgG1 format, and antigen-antibody affinity was measured using the anti-human Fc capture method with a GST fusion protein (expression host cell: HEK). The antigen-binding affinity of huAb27-9_T35 increased 3.5-fold compared to that of the parent antibody huAb27-9 (FIG. 20).

### Example 2-3: Screening of combinatorial library

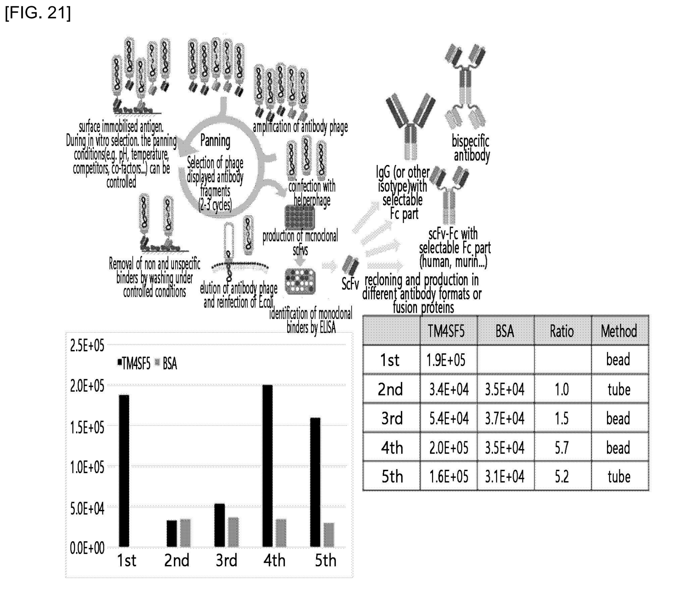

A combinatorial library of CDR-H1 and CDR-H2, where mutations were mainly observed among clones selected from the targeted library, was constructed at a diversity level of 1.4 × 10⁹. Screening was performed through a panning process, and enrichment was observed starting from the fourth round of panning (FIG. 21).

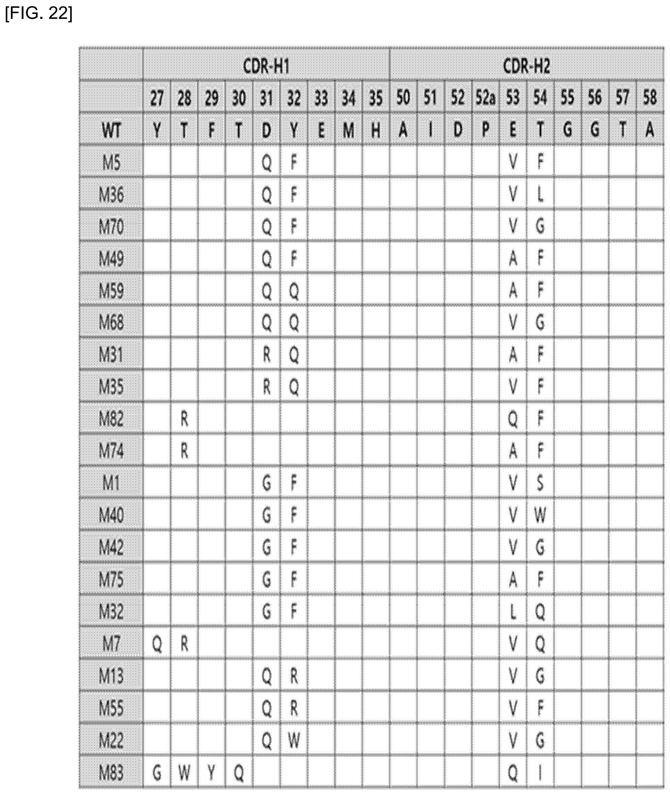

Sequence analysis of the selected clones revealed that mutations mainly appeared at positions 31 and 32 of CDR-H1 and at positions 53 and 54 of CDR-H2. The selected clones were converted to IgG1 and assessed for binding affinity (FIG. 22).

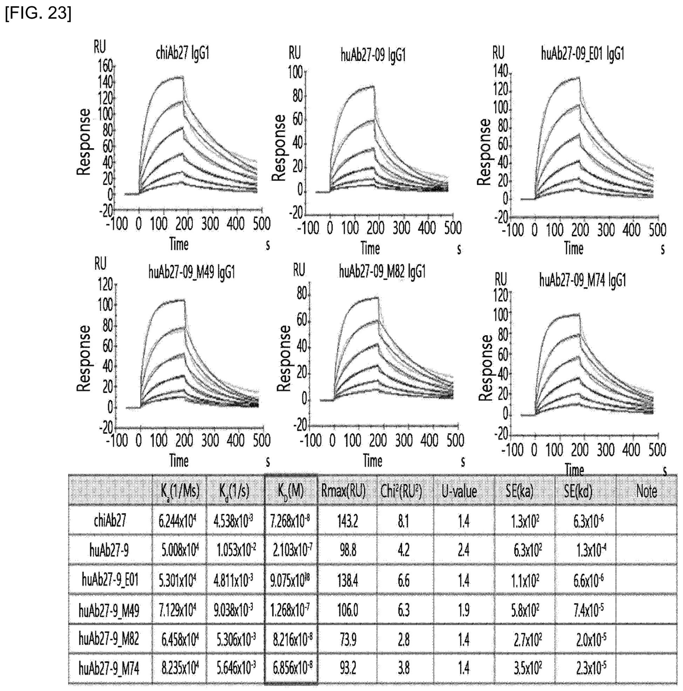

The selected antibodies were converted to the IgG1 format, transiently expressed in HEK293 cells, and antigen-antibody affinity was measured using the anti-human Fc capture method with a GST fusion protein (expression host cell: *E. coli*)*.* The antigen-binding affinities of huAb27-9_E01, M49, M82, and M74 were increased compared to that of the parent antibody huAb27-9 (FIG. 23). Antibody huAb27-9_M49, huAb27-9_M82, and huAb27-9_M74 were designated as S3, S13, and S43, respectively.

### Example 2-4: Second screening of targeted library

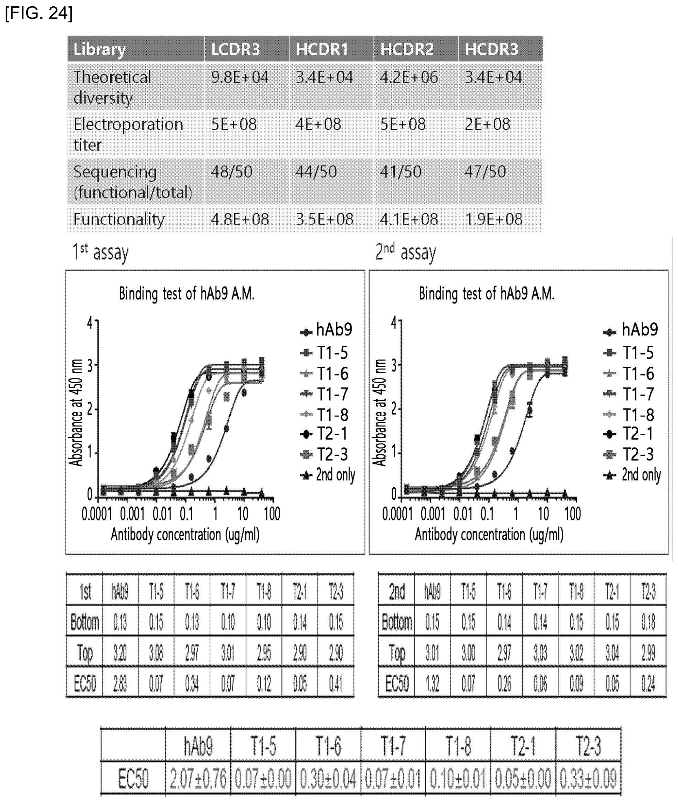

Four sub-libraries (Fab format) were constructed by introducing diversity into the HCDR1, HCDR2, HCDR3, and LCDR3 regions (based on Kabat) of the huAb27-9 antibody. Each sub-library was designed using primers (NNS) designed to allow up to four consecutive amino acid changes. For each round of biopanning, clones with improved affinity were selected by using the method of decreasing the antigen amount or increasing the number of washing steps. As a result of ELISA and sequence analysis of the output clones, a total of 15 unique clones were obtained (3 from the HCDR1 sub-library, 11 from the HCDR2 sub-library, and 1 from the HCDR3 sub-library). ELISA binding analysis normalized to expression levels showed that six clones exhibited improved affinity compared with huAb27-9 (FIG. 24).

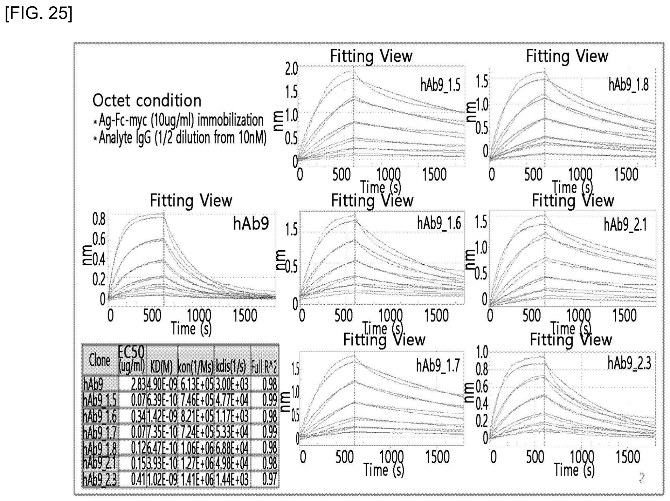

The binding affinity of the six antibodies produced as IgG antibodies against hEC2-Fc-myc antigen was analyzed by ELISA and Octet, and as a result, all clones showed improved binding compared with huAb27-9 (FIG. 25).

The T2-1 clone demonstrated a 41.4-fold improvement based on ELISA (EC₅₀ value) and a 12.6-fold improvement based on Octet (K_{D} value) relative to huAb27-9. The T1-5 clone demonstrated a 7.7-fold improvement based on Octet (K_{D} value). All six clones contained four amino acid mutations at the same positions within HCDR2.

### Example 3: Characterization and efficacy evaluation of selected antibody clones

### Example 3-1: Antibody clones and additional selection

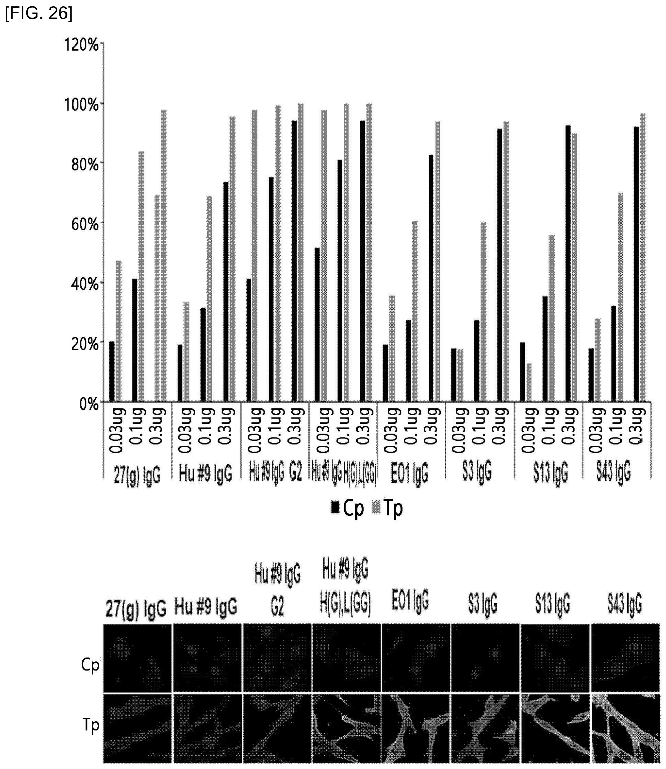

To improve the binding affinity of Hz#9 (huAb27-9) IgG, affinity maturation was performed using targeted mutagenesis and untargeted mutagenesis, and five antibodies-EO1, T35, S3, S13, and S43-were selected by measuring the target-binding abilities using a BIACORE instrument. The cell-surface binding abilities of the selected antibodies were measured by FACS and IF assays, and the results are shown in FIG. 26.

In the FACS assay, similar to Hz#9 IgG, EO1 and S43 showed differences in binding affinities to Cp and Tp cells even at 0.03 µg, although their binding affinities were slightly weaker than that of Hz#9 H(G)L(GG). S3 and S13 did not show differences in binding to Cp and Tp cells at 0.03 µg.

In the IF assay, unlike the FACS results, the constructed antibodies showed substantially greater binding to the cell surface than Hz#9 IgG. Meanwhile, EO1 and S3 exhibited binding comparable to Hz#9 H(G)L(GG), whereas S13 and S43 showed even greater binding than Hz#9 H(G)L(GG). T35 showed markedly reduced binding in both FACS and IF assays.

Accordingly, among the five antibodies, EO1 and S43 were selected as candidate antibodies.

### Example 3-2: Evaluation of target-binding ability of antibodies with additional glycine in the elbow region

To further enhance the target-binding abilities of the selected EO1 and S43 antibodies, antibodies were constructed by adding "G" or "GG" amino acids into the elbow regions of the IgG heavy chain and light chain.

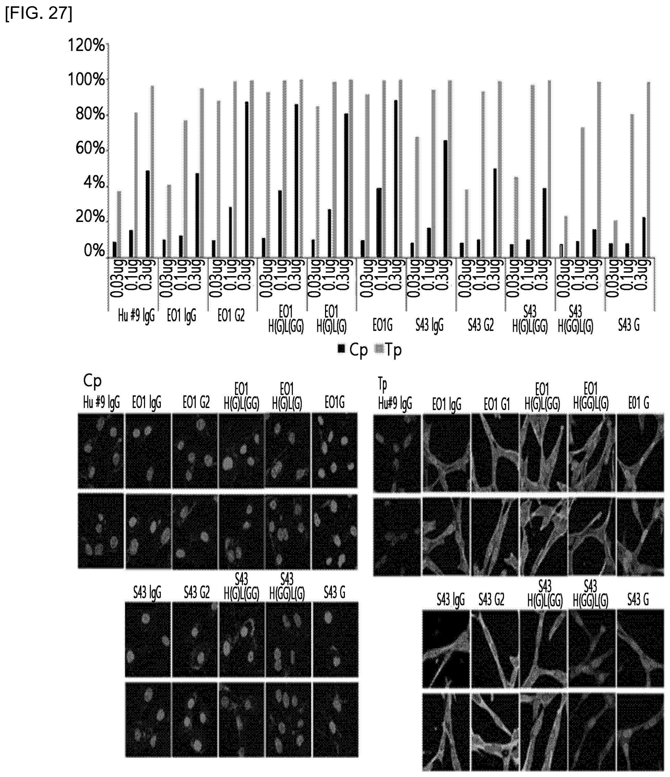

To evaluate the binding affinities of the constructed antibodies, FACS and IF assays were performed to measure the ability to bind to the target on the cell surface, and the results are shown in FIG. 27.

In the FACS assay, the order of cell-surface target-binding ability was EO1 H(G)L(GG) >= EO1 (G2) > EO1 (G) > S43 IgG > EO1 H(GG)L(G) > S43 (G2) > S43 H(G)L(GG) > Hz#9 IgG > EO1 IgG > S43 (G) > S43 H(GG)L(G). In addition, EO1 exhibited a substantial increase in binding upon addition of "G" or "GG," whereas S43 showed decreased binding ability compared with the original antibody.

In the IF assay, the order of binding was S43 (G2) > S43 = S43 H(G)L(GG) > EO1 H(G)L(GG) > EO1 (G2) > EO1 H(GG)L(G) > EO1 (G) > S43 (G) > EO1 > S43 H(GG)L(G) > Hz#9 IgG. As in the FACS results, EO1 exhibited increased binding when "G" or "GG" was added, while S43 showed increased binding only in the S43 (G2) variant. In the FACS assay, EO1 H(G)L(GG) showed the highest binding ability, whereas, in the IF assay, S43 (G2) showed the highest binding ability.

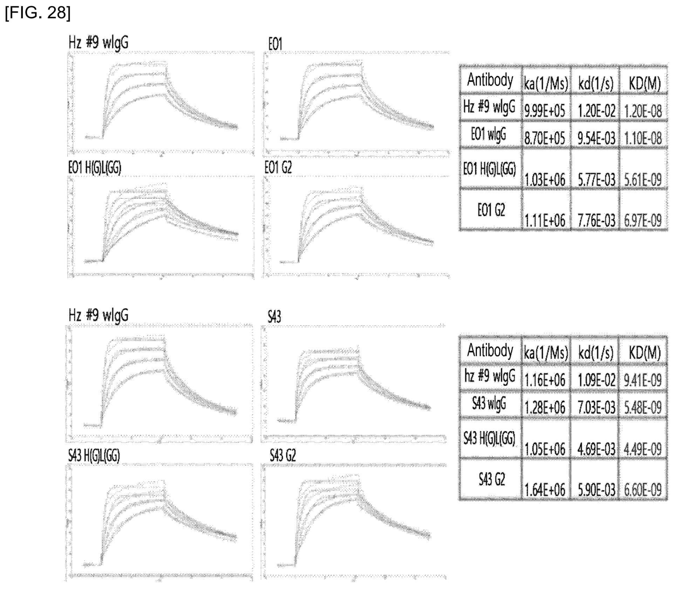

To evaluate the target-binding abilities of the constructed antibodies, K_{D} values were measured using a BIACORE instrument, and the results are shown in FIG. 28.

Based on the BIACORE analysis results, the order of binding affinity was S43 (G2) > EO1 H(G)L(GG) wIgG = S43 > Hz#9 H(G)L(GG) > EO1 > Hz#9 IgG, and EO1 H(G)L(GG) exhibited approximately two-fold improvement in affinity compared to that of EO1, while S43 (G2) exhibited approximately 1.5-fold reduction in affinity compared to that of S43.

### Example 3-3: Internalization evaluation

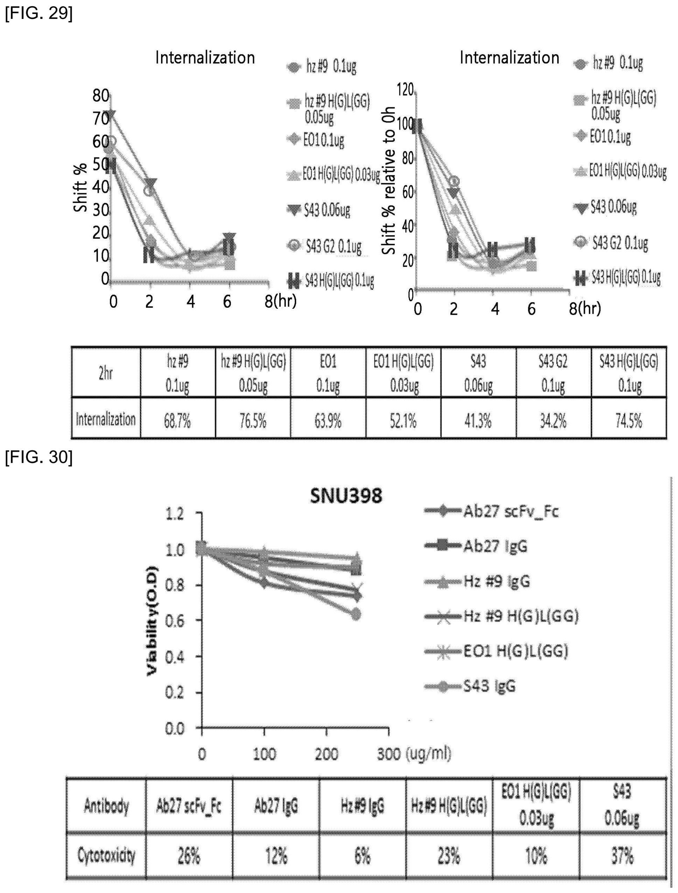

TM4SF5 antibodies exhibit internalization over time after binding to a target on the cell surface. Therefore, to compare the internalization of antibodies, the remaining amount of antibody on the surface of Tp cells was analyzed by FACS, and the results are shown in FIG. 29.

The constructed antibodies showed internalization in the order Hz#9 H(G)L(GG) 0.05 µg > S43 H(G)L(GG) 0.1 µg > Hz#9 IgG 0.1 µg > EO1 IgG 0.1 µg > EO1 H(G)L(GG) 0.03 µg > S43 IgG 0.06 µg > S43 (G2) 0.1 µg.

Overall, Hz#9 IgG H(G)L(GG), EO1 IgG H(G)L(GG), and S43 IgG exhibited significantly improved target-binding affinity, cell-surface binding, and internalization compared to those of Hz#9 IgG. Therefore, these antibodies were selected for subsequent experiments.

The results of Examples 3-2 and 3-3 are summarized in the table below.

**[Table 6]**

| Experimental method | Resu.s |
|---|---|
| FACS | Hz #9 H(G)L(GG) > EO1 H(G)L(GG) > S43 IgG > S43 (G2) > Hz #9 IgG > EO1 IgG |
| IF | S43 (G2) > S43 IGG > EO1 H(G)L(GG) > EO1 IgG > Hz #9 H(G)L(GG) > Hz #9 IgG |
| BIACORE | S43 (G2) > EO1 H(G)L(GG) = S43 IgG > Hz #9 Hz #9 H(G)L(GG) > EO1 IgG > Hz #9 IgG |
| Internalization | Hz #9 H(G)L(GG) 0.005ug > S43 H(G)L(GG) 0.1ug > hz #9 IgG 0.1ug > EO1 IgG 0.1ug > EO1 H(G)L(GG) 0.03ug > S43 IgG 0.06ug > S43 (G2) 0.1ug |

### Example 3-4: Evaluation of tumor growth inhibition activity in vitro

To compare the ability of the selected TM4SF5 antibodies to inhibit the growth of the SNU398 liver cancer cell line, humanized antibodies were treated at 100 µg/mL and 250 µg/mL to assess cytotoxicity. The results are shown in FIG. 30.

The results demonstrated that treatment with antibodies at 250 µg/mL clearly reduced cell growth, with growth inhibition occurring in the order S43 IgG > Ab27 scFv-Fc > Hz#9 H(G)L(GG) > Ab27 IgG = EO1 H(G)L(GG) > Hz#9 IgG.

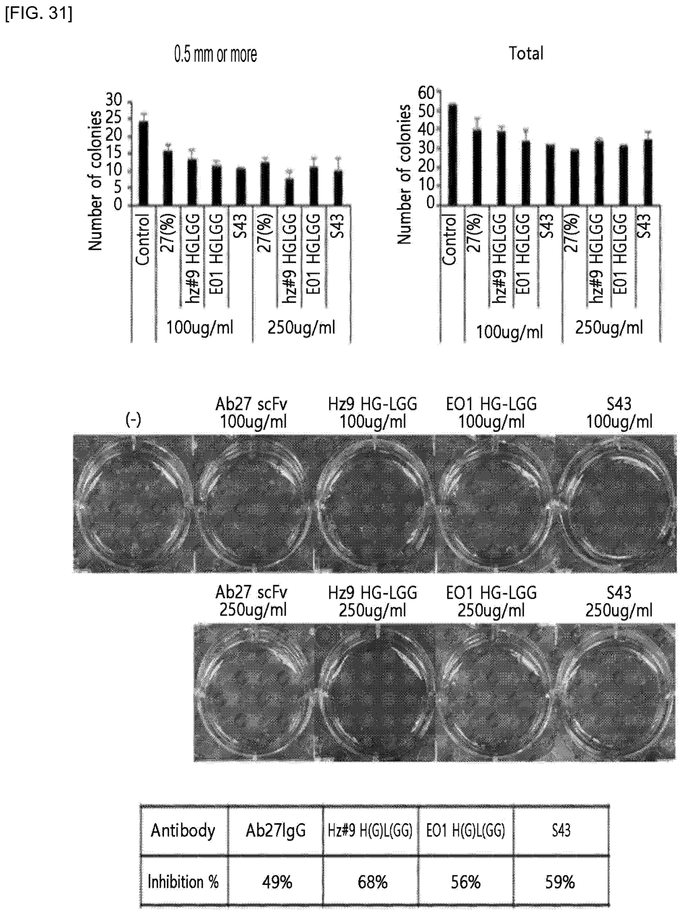

TM4SF5 antibodies exhibit inhibition of tumor formation of SNU398, a liver cancer cell line. To evaluate whether the selected antibodies are capable of inhibiting tumorigenic ability, a soft agar assay was performed using SNU398 cells, and the results are shown in FIG. 31.

Treatment with antibodies significantly reduced the number of SNU398 colonies larger than 0.5 mm, and colony formation was inhibited in the order Hz#9 H(G)L(GG) > S43 IgG > EO1 H(G)L(GG) > Ab27 IgG.

### Example 3-5: Evaluation of tumor inhibition activity in vivo

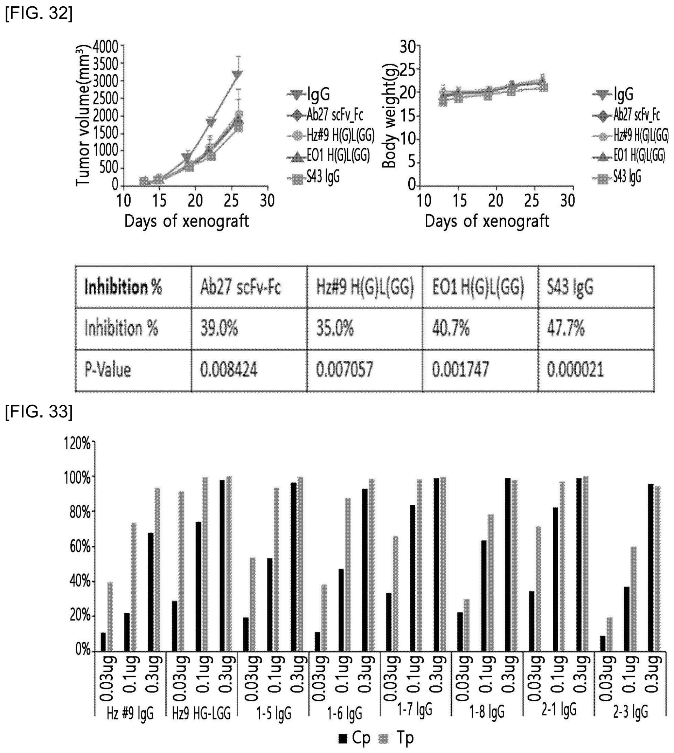

To confirm the tumor inhibition properties of the antibodies selected *in vitro,* an SNU398 cell xenograft study was conducted in nude mice. When tumor volume reached about 100 mm³, human IgG, Ab27 scFv-Fc (300 µg), Hz#9 H(G)L(GG), EO1 H(G)L(GG), and S43 IgG (300 µg/mouse) were administered intraperitoneally three times per week (seven total injections). The results are shown in FIG. 32.

As a result, in the SNU398 xenograft model, the tumor inhibition rates were Ab27 scFv-Fc IR 39%, Hz#9 H(G)L(GG) IR 35%, EO1 H(G)L(GG) IR 41%, and S43 IgG IR 48%. The tumor inhibition effect was in the order S43 IgG > EO1 H(G)L(GG) > Ab27 scFv-Fc > Hz#9 H(G)L(GG). Thus, among the selected antibodies, S43 exhibited the greatest tumor inhibition ability, with no significant change in body weight.

### Example 4: Characterization and efficacy evaluation of selected antibody clones

Six clones-1-5, 1-6, 1-7, 1-8, 2-1, and 2-3-selected from Example 2-4 were constructed in IgG format and evaluated by ELISA and BLI (Octet) analyses.

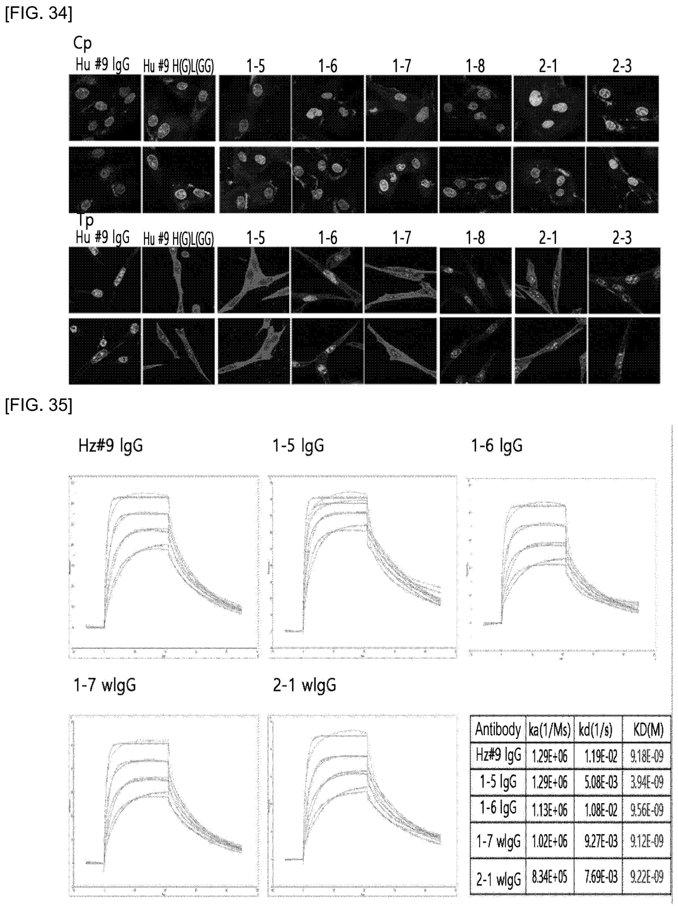

The selected antibodies were assessed for cell-surface target-binding ability using FACS and IF assays. The results are shown in FIGS. 33 and 34.

In the FACS assay, the binding ability was in the order Hz#9 H(G)L(GG) > 2-1 > 1-7 > 1-5 > 1-6 > 1-8 > Hz#9 IgG > 2-3. In the IF assay, the order was 1-5 > 1-7 > Hz#9 H(G)L(GG) > 2-1 > 1-6 = 2-3 > 1-8 > Hz#9 IgG. As a result, 2-3 was excluded from candidate selection because it showed weaker binding ability than Hz#9 IgG in the FACS assay.

To measure the TM4SF5 target-binding ability of the selected antibodies, K_{D} values were measured using a BIACORE instrument, and the results are shown in FIG. 35. Based on the BIACORE analysis results, the binding ability was observed in the order 1-5 IgG > Hz#9 IgG = 1-6 IgG = 1-7 IgG = 2-1 IgG, with 1-5 IgG showing significantly higher binding ability than the other antibodies.

Overall, based on the results of the FACS, IF, and BIACORE analyses, antibodies 1-5 and 2-1 among antibodies 1-5, 1-6, 1-7, 2-1, and 2-3 were selected for further experiments due to their significantly enhanced target-binding ability compared with Hz#9 IgG.

### Example 5: Comparative evaluation of selected clones

### Example 5-1: Purification and SPR analysis

For *in vivo* efficacy evaluation, the major antibodies selected were produced on a large scale by transient expression in mammalian cells (outsourced to Osong Advanced Medical Industry Promotion Foundation).

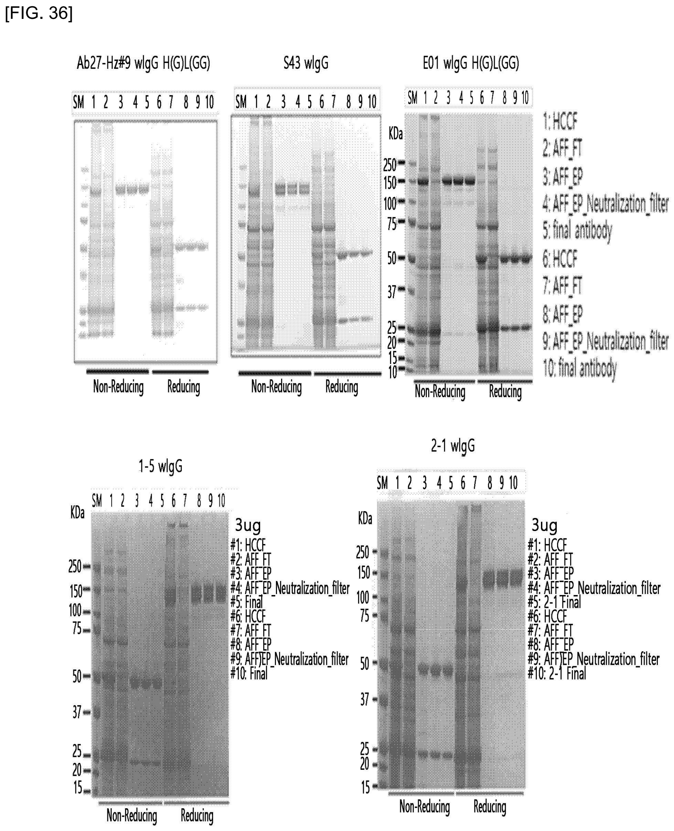

Using the Expi-CHO-S system, 1 L to 3 L of culture after transient expression was obtained per clone, and the culture supernatant was purified by affinity chromatography (AKTA Avant 150, MabSelect SuRe column) (FIG. 36). Approximately 100 mg to 200 mg/species was obtained. The final buffer was 1× PBS, pH 7.2. Endotoxin testing showed that the endotoxin level of E01 was 5.56 EU/Ab mg.

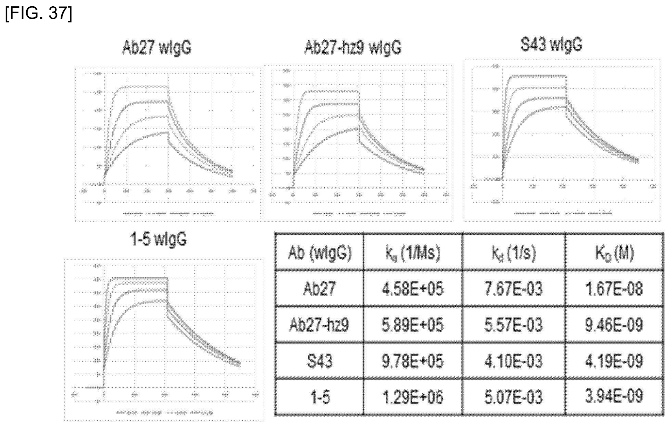

The SPR analysis results of the selected antibodies are shown in FIG. 37. The K_{D} values of S43 (4.19 nM) and 1-5 (3.94 nM) were improved by approximately four-fold compared with the chimeric Ab27 (16.7 nM) and approximately 2.4-fold compared with the humanized Ab27-hz9 (9.46 nM).

### Example 5-2: Cell-surface target-binding ability analysis

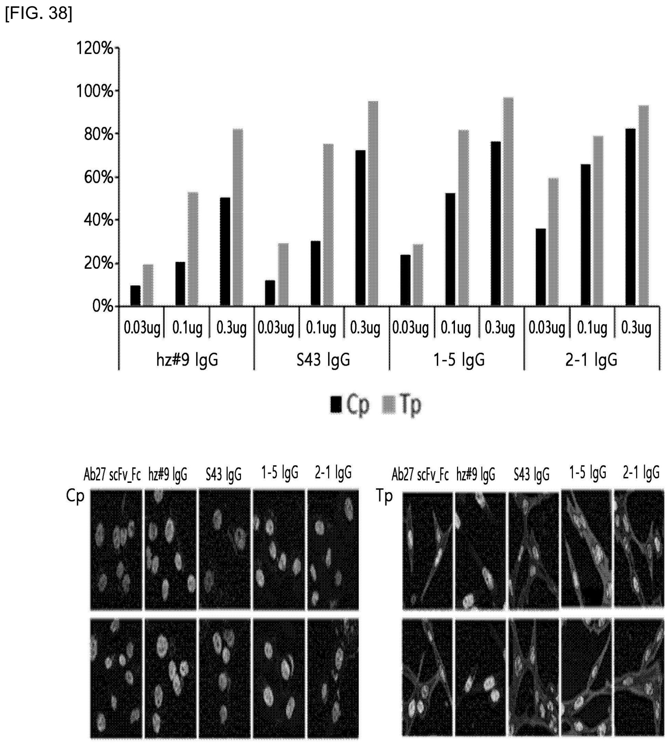

To analyze the cell-surface target-binding abilities of S43, 1-5, and 2-1 selected in the foregoing examples, FACS and IF assays were performed. The results are shown in FIG. 38.

In the FACS assay, the order of binding ability was 2-1 = 1-5 > S43 > Hz#9 IgG. However, for 2-1, the shift difference between Cp and Tp cells was markedly smaller than that of the other antibodies, suggesting that its binding specificity to TM4SF5 is weaker than that of the other antibodies. In the IF assay, the order of binding ability was 1-5 IgG > 2-1 IgG = Ab27 scFv-Fc = S43 IgG > Hz#9 IgG.

### Example 5-3: Internalization analysis

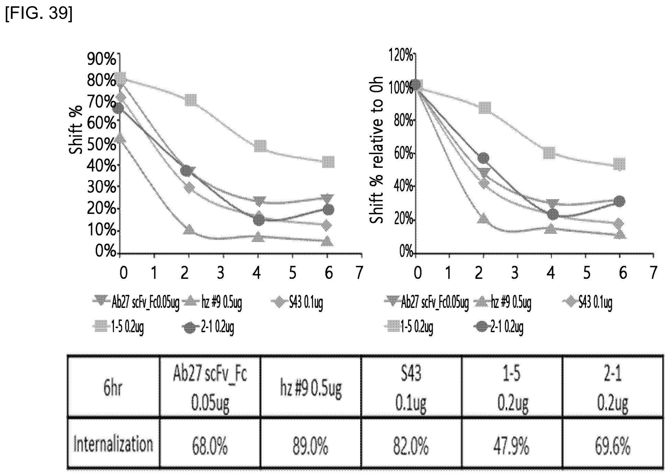

To compare the internalization of humanized TM4SF5 antibodies over time after binding to targets on the cell surface, previously selected S43 was used as a reference. Antibodies were bound to the surface of Tp cells, and the remaining antibodies were analyzed at 0, 2, 4, and 6 hours by FACS. The results are shown in FIG. 39.

Based on fluorescence measurements of residual antibodies on the cell membrane, internalization occurred in the order Hz#9 IgG 0.5 µg > S43 0.1 µg > 2-1 IgG 0.2 µg = Ab27 scFv-Fc 0.05 µg > 1-5 IgG 0.2 µg. Although 1-5 showed high cell-surface binding ability, it exhibited significantly lower internalization than the other antibodies.

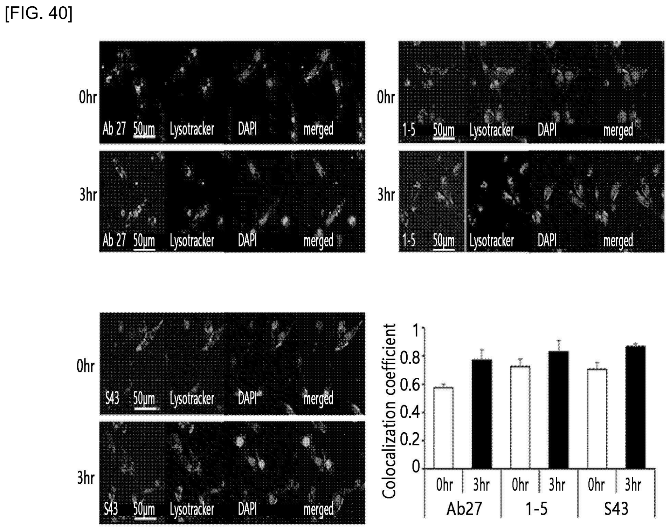

To track whether TM4SF5 antibodies migrate to lysosomes after internalization, co-IF assays were performed using LysoTracker, a lysosome marker. Antibodies were bound to SNU449Tp cells at 50 µg/mL for 3 hours, followed by antibody removal and internalization for 0 or 3 hours. LysoTracker staining was performed at 200 nM for 2 hours, and fluorescence was measured using confocal microscopy. The results are shown in FIG. 40.

The results confirmed co-localization of antibodies with LysoTracker, and co-localization increased after 3 hours of internalization compared with 0 hours. The degree of co-localization of antibody with LysoTracker in the cells was in the order S43 > 1-5 > Ab27. However, even in the 0-hour internalization cells, the antibodies became internalized during the LysoTracker staining process, showing a tendency for high co-localization.

### Example 5-4: Confirmation of in vitro tumor inhibition effect

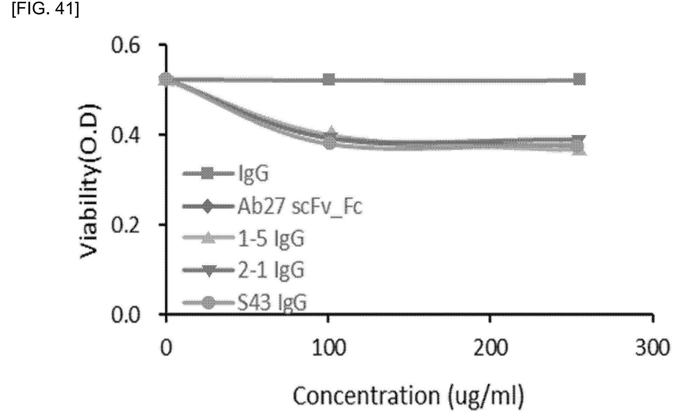

To compare the ability to inhibit the growth of the SNU398 liver cancer cell line, the antibodies were treated at 100 µg/mL and 250 µg/mL to assess cytotoxicity. The results are shown in FIG. 41.

When treating the antibodies at 250 µg/mL, Ab27 scFv-Fc, 1-5 IgG, 2-1 IgG, and S43 IgG all showed approximately 30% reduction in cell growth.

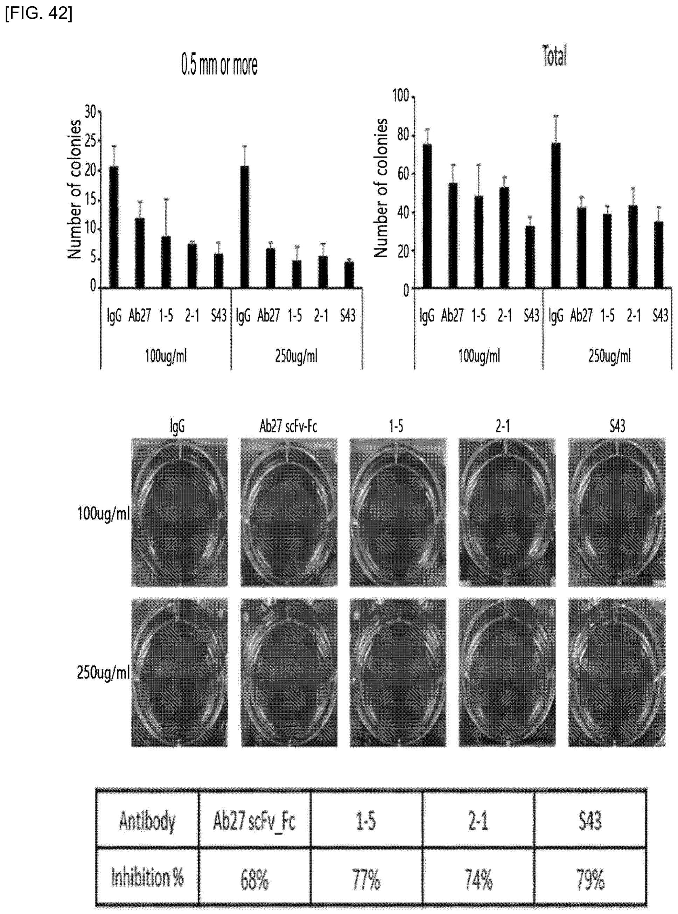

In addition, to determine whether the selected antibodies can reduce tumorigenic ability *in vitro,* a soft agar assay was performed. The results are shown in FIG. 42.

Treatment with antibodies significantly reduced the number of SNU398 colonies larger than 0.5 mm, and colony formation was inhibited in the order S43 IgG > 1-5 > 2-1 > Ab27.

### Example 5-5: Stability evaluation

To assess the stability of the selected antibodies, their concentration changes were measured after storage at 4°C for 39 days.

**[Table 7]**

| | Original concentration (mg/mL) | Concentration after 39 days of refrigeration (mg/mL) | Ratio% |
|---|---|---|---|
| hz#9 IgG | 0.577 | 0.445 | 77.1 |
| S43 IgG | 2.015 | 1.912 | 94.9 |
| 1-5 IgG | 2.059 | 1.859 | 90.3 |
| 2-1 IgG | 2.074 | 2.014 | 97.1 |

While Hz#9 IgG exhibited approximately 23% reduction in concentration after 39 days, S43 IgG, 1-5 IgG, and 2-1 IgG all showed less than 10% reduction, indicating that the stability of the affinity-matured antibodies was significantly improved compared with Hz#9 IgG.

### Example 5-6: Verification of in vitro effect of combination administration

The effects of the combination therapeutic agents sorafenib and cetuximab on colorectal cancer cell lines HT-29 and HCT116 were tested *in vitro.*

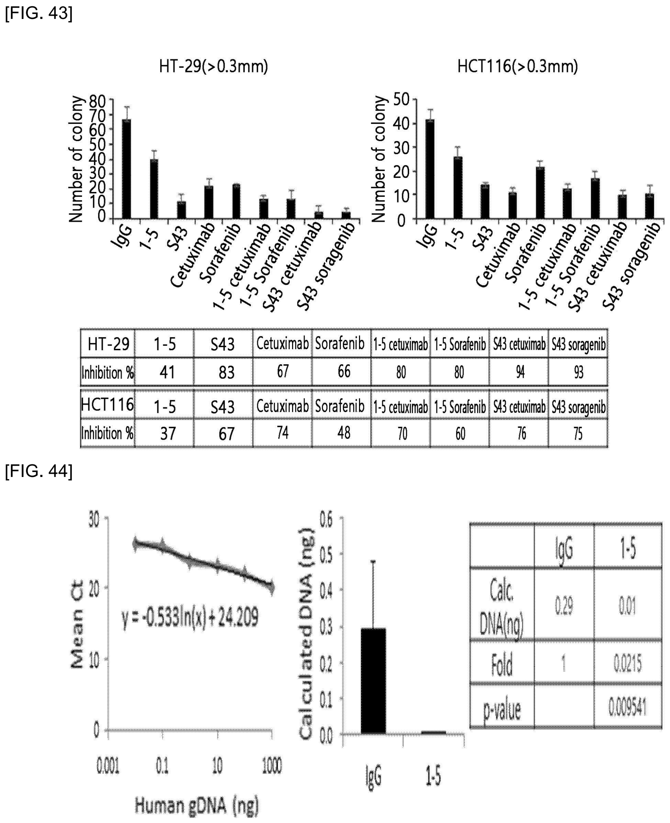

Soft agar assays were performed to evaluate tumorigenesis inhibition, using the following treatments: 1-5 IgG 100 µg/mL alone; S43 IgG 100 µg/mL alone; cetuximab 100 µg/mL alone; sorafenib 500 nM alone; co-administration of 1-5 and cetuximab; co-administration of 1-5 and sorafenib; co-administration of S43 and cetuximab; and co-administration of S43 and sorafenib. Colony formation after 14 days was analyzed. The results are shown in FIG. 43.

The results showed that 1-5 and S43 exhibited tumor inhibition effects on colorectal cancer cell lines, and it was confirmed that co-administration with sorafenib or cetuximab enhanced tumor inhibition .

### Example 5-7: Confirmation of in vivo tumor metastasis inhibition effect

To evaluate whether the antibodies reduce the metastatic ability of liver cancer cell lines through blood vessels in an *in vivo* early metastasis model, 300 µg of 1-5 IgG was administered intraperitoneally to nude mice. SNU398 cells were injected intravenously via the tail vein, and circulating tumor cell (CTC) levels were measured by qPCR after 24 hours.

The results showed that 1-5 IgG effectively reduced CTC levels, demonstrating approximately 97.8% reduction.

### Example 5-8: Confirmation of in vivo tumor inhibition effect

### Example 5-8-1: Evaluation of effect in the SNU398 xenograft model

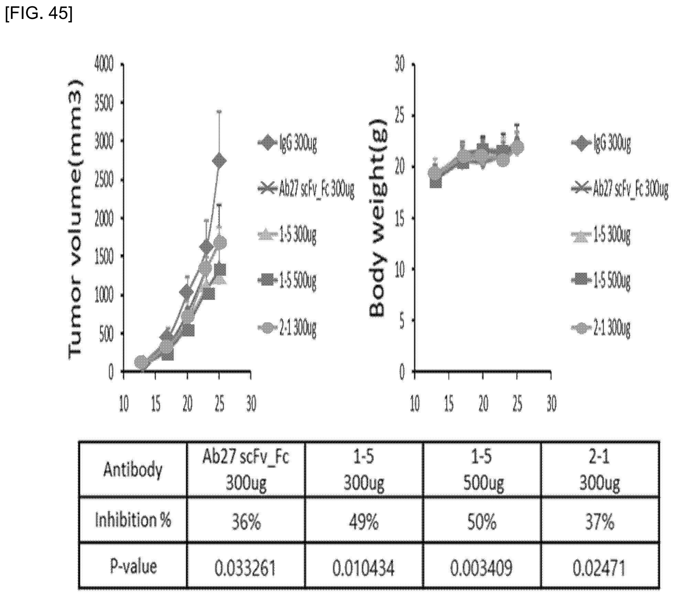

To verify whether the selected antibodies inhibit the liver cancer cell line SNU398, a xenograft study was conducted in nude mice. When tumor volume reached approximately 100 mm³, human IgG 300 µg/mouse, Ab27 scFv-Fc 300 µg/mouse, 1-5 IgG 300 or 500 µg/mouse, and 2-1 IgG 300 µg/mouse were administered intraperitoneally three times per week (six total injections). The results are shown in FIG. 45.

In the xenograft model, the tumor inhibition rates were as follows: Ab27 scFv-Fc 36%, 1-5 IgG 300 µg/mouse 49%, 1-5 IgG 500 µg/mouse 50%, and 2-1 IgG 300 µg/mouse 37%. Tumor inhibition effects were in the order 1-5 IgG > 2-1 IgG > Ab27 scFv-Fc. Increasing the 1-5 IgG dose to 300 µg/mouse or more did not produce significant differences in tumor inhibition ability, indicating 300 µg/mouse as the saturation dose. No significant changes in body weight by the antibody injections were observed.

### Example 5-8-2: Evaluation of effect in the SNU449 T7 xenograft model

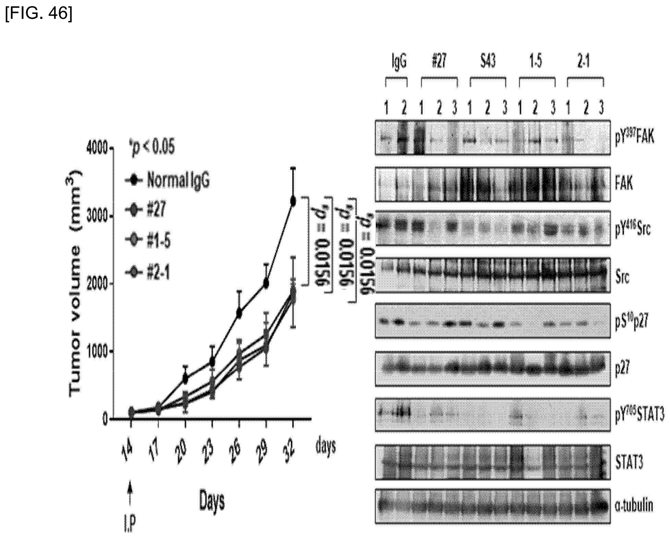

The antitumor efficacy of 1-5 and 2-1 was evaluated in the SNU449 T7 xenograft model. T7 cells (1×10⁶) were subcutaneously injected, and when tumor size reached 100 mm³ after two weeks, the antibodies were administered at 300 µg/mouse intraperitoneally twice per week. The results are shown in FIG. 46.

Both 1-5 and 2-1 showed tumor growth inhibition efficacy comparable to that of Ab27.

Western blot analysis of tumor lysates from the previous xenograft studies and the present study showed the following results (FIG. 46): inhibition of phosphorylation of FAK (moderate), c-Src, and STAT3 by S43; inhibition of p27 and STAT3 phosphorylation by 1-5; and inhibition of FAK and p27 phosphorylation by 2-1.

### Example 5-8-3: Evaluation of effect in the SNU449 T7 xenograft model

To assess tumor inhibition in the colorectal cancer cell line HT-29, a xenograft study in nude mice was conducted.

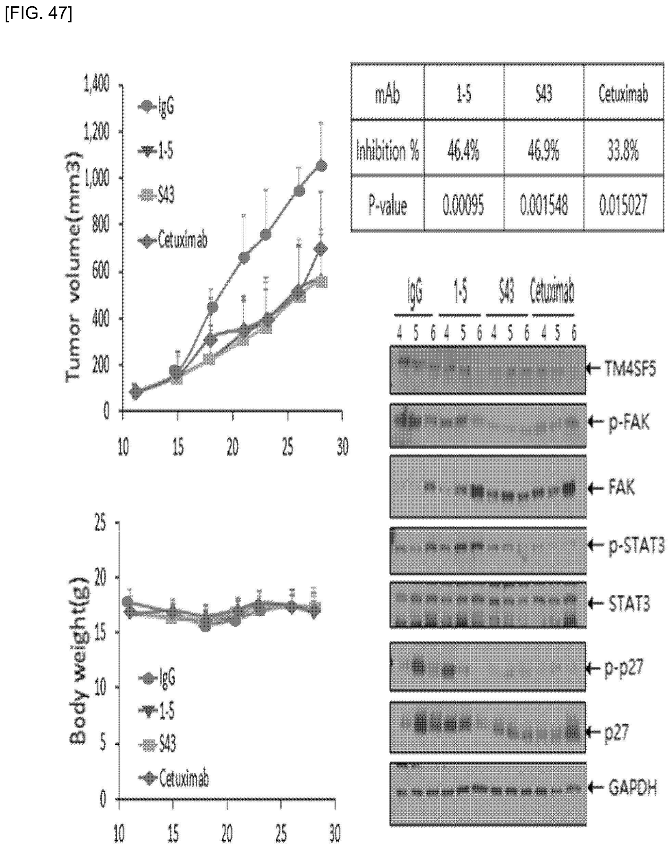

When tumors reached approximately 100 mm³, human IgG, 1-5 IgG, S43 IgG, and cetuximab (300 µg/mouse) were administered intraperitoneally three times per week (eight total injections). The results are shown in FIG. 47.

In the xenograft model, the inhibition rates were: 1-5 (46.4%), S43 (46.9%), and cetuximab (33.8%). Tumor inhibition followed the order S43 IgG > 1-5 IgG > cetuximab. Both 1-5 and S43 showed tumor inhibition comparable to or better than cetuximab, with no significant body-weight changes. The phosphorylation levels of FAK, STAT3, and p27 were reduced by S43. The effect of 1-5 was relatively moderate.

### Example 5-9: Evaluation of in vivo tumor targeting ability

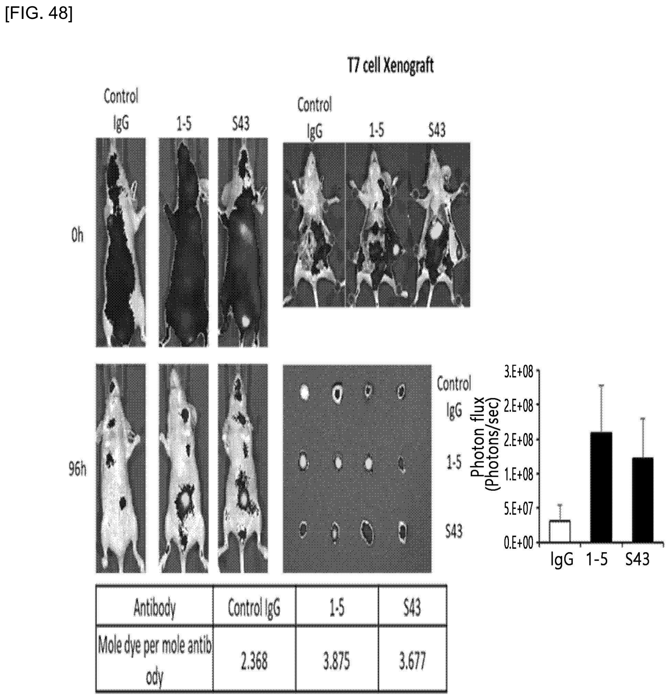

To analyze tissue distribution of the selected antibodies in a mouse model, fluorescent dye-conjugated antibodies were prepared. T7 cells, a TM4SF5-expressing clone derived from the liver cancer cell line SNU449, were subcutaneously injected into nude mice. When tumors reached approximately 125 mm³, fluorescent dye-conjugated antibodies were administered via the tail vein, and fluorescence *in vivo* was measured by IVIS. The results are shown in FIG. 48.

While human IgG was mostly degraded after 96 hours, 1-5 and S43 were clearly distributed in tumors. Both antibodies showed excellent tumor-targeting ability. In particular, 1-5 was distributed more in tumors compared with S43, showing slightly superior *in vivo* tumor targeting ability.

The sequences of the selected antibodies S43, 1-5, 2-1, and E01 are as described in SEQ ID NOS: 14 to 50. CDRs defined according to IMGT are as described in Table 12. The VL and LCDR sequences of antibodies S43, 1-5, and 2-1 were identical to those of humanized antibody huAb27-9.

**[Table 8]**

| # | Antibody | Name | Numbering | Sequence |
|---|---|---|---|---|
| 14 | S43 | VH | | QVQLVQSGAEVKKPGASVKVSCKASGYRFTDYEMHWVRQA PGQGLEWMGAIDPAFGGTAYNQKFKGRVTMTADKSISTAY MELSRLRSDDTAVYYCARPYLGYWGQGTLVTVSS |
| 15 | | heavy chain | | QVQLVQSGAEVKKPGASVKVSCKASGYRFTDYEMHWVRQA PGQGLEWMGAIDPAFGGTAYNQKFKGRVTMTADKSISTAY MELSRLRSDDTAVYYCARPYLGYWGQGTLVTVSSASTKGP SVFPLAPSSKSTSGGTAALGCLVKDYFPEPVTVSWNSGAL TSGVHTFPAVLQSSGLYSLSSVVTVPSSSLGTQTYICNVN HKPSNTKVDKKVEPKSCDKTHTCPPCPAPELLGGPSVFLF PPKPKDTLMISRTPEVTCVVVDVSHEDPEVKFNWYVDGVE VHNAKTKPREEQYNSTYRVVSVLTVLHQDWLNGKEYKCKV SNKALPAPIEKTISKAKGQPREPQVYTLPPSRDELTKNQV SLTCLVKGFYPSDIAVEWESNGQPENNYKTTPPVLDSDGS FFLYSKLTVDKSRWQQGNVFSCSVMHEALHNHYTQKSLSL SPGK |
| 16 | | HCDR1 | Kabat | DYEMH |
| 17 | | HCDR2 | | AIDPAFGGTAYNQKFKG |
| 18 | | HCDR3 | | PYLGY |
| 19 | | HCDR1 | Chothia | GYRFTDY |
| 20 | | HCDR2 | | DPAFGG |
| 21 | | HCDR3 | | PYLGY |

**[Table 9]**

| # | Antibody | Name | Numbering | Sequence |
|---|---|---|---|---|
| 22 | 1-5 | VH | | QVQLVQSGAEVKKPGASVKVSCKASGYTFTDYEMHWVR QAPGQGLEWMGAIDPETGGTFVKRKFKGRVTMTADKSI STAYMELSRLRSDDTAVYYCARPYLGYWGQGTLVTVSS |
| 23 | | heavy chain | | QVQLVQSGAEVKKPGASVKVSCKASGYTFTDYEMHWVR QAPGQGLEWMGAIDPETGGTFVKRKFKGRVTMTADKSI STAYMELSRLRSDDTAVYYCARPYLGYWGQGTLVTVSS ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCPA PELLGGPSVFLFPPKPKDTLMISRTPEVTCVVVDVSHE DPEVKFNWYVDGVEVHNAKTKPREEQYNSTYRVVSVLT VLHQDWLNGKEYKCKVSNKALPAPIEKTISKAKGQPRE PQVYTLPPSRDELTKNQVSLTCLVKGFYPSDIAVEWES NGQPENNYKTTPPVLDSDGSFFLYSKLTVDKSRWQQGN VFSCSVMHEALHNHYTQKSLSLSPGK |
| 24 | | HCDR1 | Kabat | DYEMH |
| 25 | | HCDR2 | | AIDPETGGTFVKRKFKG |
| 26 | | HCDR3 | | PYLGY |
| 27 | | HCDR1 | Chothia | GYTFTDY |
| 28 | | HCDR2 | | DPETGG |
| 29 | | HCDR3 | | PYLGY |

**[Table 10]**

| # | Antibody | Name | Numbering | Sequence |
|---|---|---|---|---|
| 30 | 2-1 | VH | | QVQLVQSGAEVKKPGASVKVSCKASGYTFTDYEMHWVRQ APGQGLEWMGAIDPETGGTFVVSKFKGRVTMTADKSIST AYMELSRLRSDDTAVYYCARPYLGYWGQGTLVTVSS |
| 31 | | heavy chain | | QVQLVQSGAEVKKPGASVKVSCKASGYTFTDYEMHWVRQ APGQGLEWMGAIDPETGGTFVVSKFKGRVTMTADKSIST AYMELSRLRSDDTAVYYCARPYLGYWGQGTLVTVSSAST KGPSVFPLAPSSKSTSGGTAALGCLVKDYFPEPVTVSWN SGALTSGVHTFPAVLQSSGLYSLSSVVTVPSSSLGTQTY ICNVNHKPSNTKVDKKVEPKSCDKTHTCPPCPAPELLGG PSVFLFPPKPKDTLMISRTPEVTCVVVDVSHEDPEVKFN WYVDGVEVHNAKTKPREEQYNSTYRVVSVLTVLHQDWLN GKEYKCKVSNKALPAPIEKTISKAKGQPREPQVYTLPPS RDELTKNQVSLTCLVKGFYPSDIAVEWESNGQPENNYKT TPPVLDSDGSFFLYSKLTVDKSRWQQGNVFSCSVMHEAL HNHYTQKSLSLSPGK |
| 32 | | HCDR1 | Kabat | DYEMH |
| 33 | | HCDR2 | | AIDPETGGTFVVSKFKG |
| 34 | | HCDR3 | | PYLGY |
| 35 | | HCDR1 | Chothia | GYTFTDY |
| 36 | | HCDR2 | | DPETGG |
| 37 | | HCDR3 | | PYLGY |

**[Table 11]**

| # | Antibody | Name | Numbering | Sequence |
|---|---|---|---|---|
| 38 | EO1 | VH | | EVQLVQSGADVKEPGASVKVSCKASGYTFTDYEMHWVRQAP GQGLEWMGAIDPETGGTAYNQKFKGWVTMTADKSISTAYME LSRLRSDDTAVYYCTRPYLGYWGQGTLVTVSS |
| 39 | | heavy chain | | EVQLVQSGADVKEPGASVKVSCKASGYTFTDYEMHWVRQAP GQGLEWMGAIDPETGGTAYNQKFKGWVTMTADKSISTAYME LSRLRSDDTAVYYCTRPYLGYWGQGTLVTVSSASTKGPSVF PLAPSSKSTSGGTAALGCLVKDYFPEPVTVSWNSGALTSGV HTFPAVLQSSGLYSLSSVVTVPSSSLGTQTYICNVNHKPSN TKVDKKVEPKSCDKTHTCPPCPAPELLGGPSVFLFPPKPKD TLMISRTPEVTCVVVDVSHEDPEVKFNWYVDGVEVHNAKTK PREEQYNSTYRVVSVLTVLHQDWLNGKEYKCKVSNKALPAP IEKTISKAKGQPREPQVYTLPPSRDELTKNQVSLTCLVKGF YPSDIAVEWESNGQPENNYKTTPPVLDSDGSFFLYSKLTVD KSRWQQGNVFSCSVMHEALHNHYTQKSLSLSPGK |
| 40 | | HCDR1 | Kabat | DYEMH |
| 41 | | HCDR2 | | AIDPETGGTAYNQKFKG |
| 42 | | HCDR3 | | PYLGY |
| 43 | | HCDR1 | Chothia | GYTFTDY |
| 44 | | HCDR2 | | DPETGG |
| 45 | | HCDR3 | | PYLGY |
| 46 | | VL | Kabat/Cho thia | EIVLTQSPGMLSLSPGERATLSCRSSQSLVHRNGNTYLHWY QQKPGQAPRLLIYKVSNRFSGIPDRFSGSGSGTDFTLTISR LEPEDFAVYYCFQGSHIPLTFGQGTKVEIK |
| 47 | | Light chain | | EIVLTQSPGMLSLSPGERATLSCRSSQSLVHRNGNTYLHWY QQKPGQAPRLLIYKVSNRFSGIPDRFSGSGSGTDFTLTISR LEPEDFAVYYCFQGSHIPLTFGQGTKVEIKRTVAAPSVFIF PPSDEQLKSGTASVVCLLNNFYPREAKVQWKVDNALQSGNS QESVTEQDSKDSTYSLSSTLTLSKADYEKHKVYACEVTHQG LSSPVTKSFNRGEC |
| 48 | | LCDR1 | | RSSQSLVHRNGNTYLH |
| 49 | | LCDR2 | | KVSNRFS |
| 50 | | LCDR3 | | FQGSHIPLT |

**[Table 12]**

| # | Antibody | Name | Numbering | Sequence |
|---|---|---|---|---|
| 51 | Ab27-hz9 | HCDR1 | IMGT | GYTFTDYE |
| 52 | | HCDR2 | | IDPETGGT |
| 53 | | HCDR3 | | ARPYLGY |
| 54 | | LCDR1 | | QSLVHSNGNTY |
| 55 | | LCDR2 | | KVS |
| 56 | | LCDR3 | | FQGSHIPLT |
| 57 | S43 | HCDR1 | | GYRFTDYE |
| 58 | | HCDR2 | | IDPAFGGT |
| 59 | | HCDR3 | | ARPYLGY |
| 60 | 1-5 | HCDR1 | | GYTFTDYE |
| 61 | | HCDR2 | | IDPETGGT |
| 62 | | HCDR3 | | ARPYLGY |
| 63 | 2-1 | HCDR1 | | GYTFTDYE |
| 64 | | HCDR2 | | IDPETGGT |
| 65 | | HCDR3 | | ARPYLGY |
| 66 | EO1 | HCDR1 | | GYTFTDYE |
| 67 | | HCDR2 | | IDPETGGT |
| 68 | | HCDR3 | | TRPYLGY |
| 69 | | LCDR1 | | QSLVHRNGNTY |
| 70 | | LCDR2 | | KVS |
| 71 | | LCDR3 | | FQGSHIPLT |

From the foregoing, those skilled in the art to which the present invention pertains will be able to understand that the present invention may be embodied in other specific forms without modifying the technical concepts or essential characteristics of the present invention. In this regard, the foregoing embodiments are only for illustrative purposes and should not be construed as limiting the scope of the present invention. The scope of the present invention should be interpreted to include all modifications or modified forms derived from the meaning and scope of the claims as set forth below, as well as equivalent concepts, rather than from the detailed description above.

## Claims

1. A TM4SF5-binding antibody or antigen-binding fragment thereof, comprising a variable domain comprising CDRs selected from the following:
(i) a heavy chain variable domain (VH) comprising HCDR1 having an amino acid sequence of SEQ ID NO: 16, HCDR2 having an amino acid sequence of SEQ ID NO: 17, and HCDR3 having an amino acid sequence of SEQ ID NO: 18, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13;
(ii) a heavy chain variable domain (VH) comprising HCDR1 having an amino acid sequence of SEQ ID NO: 24, HCDR2 having an amino acid sequence of SEQ ID NO: 25, and HCDR3 having an amino acid sequence of SEQ ID NO: 26, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13;
(iii) a heavy chain variable domain (VH) comprising HCDR1 having an amino acid sequence of SEQ ID NO: 32, HCDR2 having an amino acid sequence of SEQ ID NO: 33, and HCDR3 having an amino acid sequence of SEQ ID NO: 34, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13; and
(iv) a heavy chain variable domain (VH) comprising HCDR1 having an amino acid sequence of SEQ ID NO: 40, HCDR2 having an amino acid sequence of SEQ ID NO: 41, and HCDR3 having an amino acid sequence of SEQ ID NO: 42, and a light chain variable domain (VL) comprising LCDR1 having an amino acid sequence of SEQ ID NO: 48, LCDR2 having an amino acid sequence of SEQ ID NO: 49, and LCDR3 having an amino acid sequence of SEQ ID NO: 50,
wherein the CDR sequences are designated according to the Kabat numbering system.

2. The TM4SF5-binding antibody or antigen-binding fragment thereof of claim 1, comprising a variable domain comprising CDRs selected from the following:
(i) a heavy chain variable domain (VH) comprising HCDR1 having an amino acid sequence of SEQ ID NO: 16, HCDR2 having an amino acid sequence of SEQ ID NO: 17, and HCDR3 having an amino acid sequence of SEQ ID NO: 18, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13;
(ii) a heavy chain variable domain (VH) comprising HCDR1 having an amino acid sequence of SEQ ID NO: 24, HCDR2 having an amino acid sequence of SEQ ID NO: 25, and HCDR3 having an amino acid sequence of SEQ ID NO: 26, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13; and
(iii) a heavy chain variable domain (VH) comprising HCDR1 having an amino acid sequence of SEQ ID NO: 32, HCDR2 having an amino acid sequence of SEQ ID NO: 33, and HCDR3 having an amino acid sequence of SEQ ID NO: 34, and a light chain variable domain (VL) comprising LCDR1 having an amino acid sequence of SEQ ID NO: 11, LCDR2 having an amino acid sequence of SEQ ID NO: 12, and LCDR3 having an amino acid sequence of SEQ ID NO: 13,
wherein the CDR sequences are designated according to the Kabat numbering system.

3. The TM4SF5-binding antibody or antigen-binding fragment thereof of claim 1, wherein the antibody or antigen-binding fragment thereof comprises:
i) a heavy chain variable domain having an amino acid sequence selected from SEQ ID NOS: 14, 22, and 30, and a light chain variable domain having an amino acid sequence of SEQ ID NO: 9; or
ii) a heavy chain variable domain having an amino acid sequence of SEQ ID NO: 38 and a light chain variable domain having an amino acid sequence of SEQ ID NO: 46.

4. The TM4SF5-binding antibody or an antigen-binding fragment thereof of claim 1, wherein the antibody is IgG or scFv-Fc.

5. A pharmaceutical composition for preventing or treating a TM4SF5-related disease, comprising the antibody or antigen-binding fragment thereof of any one of claims 1 to 4.

6. The pharmaceutical composition of claim 5, wherein the TM4SF5-related disease is selected from fatty liver, liver fibrosis, liver cancer, stomach cancer, colorectal cancer, rectal cancer, oral cancer, pharynx cancer, larynx cancer, lung cancer, colon cancer, breast cancer, cervical cancer, endometrial cancer, ovarian cancer, prostate cancer, testicular cancer, bladder cancer, kidney cancer, pancreatic cancer, bone cancer, connective tissue cancer, skin cancer, brain cancer, thyroid cancer, leukemia, Hodgkin's disease, soft tissue sarcoma, lymphoma, and multiple myeloma hematologic cancer.

7. The pharmaceutical composition of claim 5, wherein the TM4SF5-related disease is selected from fatty liver, liver fibrosis, liver cancer, stomach cancer, colorectal cancer, colon cancer, prostate cancer, pancreatic cancer, and soft tissue sarcoma.

8. The pharmaceutical composition of claim 5, wherein the pharmaceutical composition is administered in combination with a substance selected from the group consisting of 5-fluorouracil (5-FU), oxaliplatin, doxorubicin, sorafenib, and cetuximab.

9. A polynucleotide encoding the antibody or antigen-binding fragment thereof of any one of claims 1 to 4.

10. An expression vector comprising the polynucleotide of claim 9.

11. A transformant comprising the polynucleotide of claim 10 or an expression vector comprising the same.

12. A composition for diagnosing a TM4SF5-related disease, comprising the antibody or antigen-binding fragment thereof of any one of claims 1 to 4.

13. A method for providing information for diagnosis of a TM4SF5-related disease, comprising detecting a transmembrane 4 L six family member 5 (TM4SF5) protein in a biological sample through an antigen-antibody reaction using the antibody or antigen-binding fragment thereof of any one of claims 1 to 4.
